Europäisches Patentamt

European Patent Office

Office européen des brevets

(19)

(11) **EP 1 547 607 A1**

(12) **EUROPEAN PATENT APPLICATION**
published in accordance with Art. 158(3) EPC

(43) Date of publication:
**29.06.2005 Bulletin 2005/26**

(51) Int Cl.⁷: **A61K 35/74**, A61K 39/39,
A61K 39/04, A61K 39/02,
A61K 9/107, A61K 9/19,
A61K 47/06, A61K 47/10,
A61K 47/32, A61K 35/00,
C12Q 1/04, A61P 37/04

(21) Application number: **03766694.8**

(22) Date of filing: **01.08.2003**

(86) International application number:
**PCT/JP2003/009801**

(87) International publication number:
**WO 2004/012751 (12.02.2004 Gazette 2004/07)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **02.08.2002 JP 2002226181
02.08.2002 JP 2002226196**

(71) Applicant: **Sumitomo Pharmaceuticals Company,
Limited
Osaka-shi, Osaka 541-8510 (JP)**

(72) Inventors:
• **NOMURA, Takehiko
Toyonaka-shi, Osaka 561-0802 (JP)**
• **SUNAGAWA, Makoto
Itami-shi, Hyogo 664-0875 (JP)**
• **UENISHI, Yuko
Toyonaka-shi, Osaka 561-0803 (JP)**

(74) Representative: **VOSSIUS & PARTNER
Siebertstrasse 4
81675 München (DE)**

(54) **BACTERIAL CELL WALL SKELETON COMPONENT PREPARATION**

(57)    Novel preparations comprising bacterial cell wall skeleton components and determinations of bacterial cell wall skeleton components are provided.

The invention comprises a paste consisting of bacterial cell wall skeleton components (bacteria-CWS) and an oil, and make it possible to provides a paste comprising bacteria-CWS wherein the paste has a viscosity of 0.2 to 0.7 poise (25 °C), an emulsion comprising the same, and a lyophilized formulation. The invention also make it possible to provides evaluation of a pharmaceutical composition comprising bacterial cell wall skeleton components as an effective ingredient, and an intermediate material thereof, assay of their strength, and identification of an effective ingredient by determining the long-chain fatty acid comprised in the bacterial cell wall skeleton components, or a derivative thereof.

**Description**

TECHNICAL FIELD

The invention relates to lyophilized formulations comprising bacterial cell wall skeleton components (bacteria-CWS) and intermediate materials in the production thereof, and processes for preparation of the lyophilized formulations and the intermediate materials thereof, as well as processes for analyzing a medical product comprising a bacteria-CWS, for example, of Mycobacterium and Nocarida as an effective ingredient.

BACKGROUND ART

It has been acknowledged that dead bacteria, and bacterial cell wall skeleton components of bacteria (hereinafter abbreviated as bacteria-CWS) have an immunopotentiating activity, and exhibit an anti-tumor effect for example in experimental cancer cell lines using animal models and in cancer immunotherapy for human.

Further, it has been acknowledged that administration of an oil-in-water emulsion formulation, which is prepared by dispersing and emulsifying bacterial cell wall skeleton components in an oil, extremely enhances anti-cancer effects via its immunopotetiating activity. For example, it has been reported that cancer immunotherapy using an oil-in-water emulsion of bacterial cell wall skeleton of Mycobacterium bovis bacillus Calmette-Guerin (hereinafter referred to as BCG-CWS) gave an excellent result in cancer therapy for human (Pro. Japan Acad., 70, Ser. B 205-209 (1994), Pro. Japan Acad., 74, Ser. B20550-55 (1998)). Such an oil-in-water emulsion formulation can be prepared, for example, by adding water containing a surfactant to the paste obtained by dispersing and emulsifying a bacteria-CWS in an oil (J. Nat. Cancer Inst. 48, 831-835 (1972), J. Bacteriol, 92, 869-879 (1966), Gann, 69, 619-626 (1978)).

In general, an oil-in-water emulsion comprising bacteria-CWS is extremely unstable to generate insoluble aggregates in a few days. Such a tendency is especially pronounced in cases that oil contents are low. On the other hand, oil-in-water emulsion formulations with higher oil contents may put a much burden on living bodies and also provide side effects. Therefore, only a required amount of an oil-in-water emulsion formulation is now manually prepared just before use in clinical practice. However, it is difficult to prepare formulations constantly having uniform qualities just before use by such manual operations. Thus, re-suspension of a lyophilized formulation with high storage-stability before use, which is prepared by lyophilizing an oil-in-water emulsion, into an oil-in-water emulsion formulation by adding an aqueous solvent such as distilled water for injection to the lyophilized formulation is preferable from the viewpoint of availability for clinical use and storage stability of the formulations. Accordingly, there exists a need to provide a stable oil-in-water emulsion formulation and a lyophilized formulation thereof, which can be put into practical use as medical products and can be constantly prepared.

In order to place a formulation comprising bacteria-CWS as an effective ingredient on commercial market, assay of biological strength and identification of effective ingredients are required in addition to the preparation of such a formulation. In general, medical products comprising a chemical substance as an effective ingredient can be evaluated for identity and content of the ingredient by means of determination using chemical analysis.

However, bacteria-CWS which is a biopolymer having a three-layer structure containing long-chain fatty acids such as mycolic acid, sugar chains and peptide glycans is insoluble in water and organic solvents, and therefore, it cannot be determined by methods such as HPLC, GC and the like which are usually used for analysis of chemical substances.

Bacteria have different constituents depending on genera, species and strains. Methods for analysis of mycolic acid which characteristically exists in Mycobacterium such as tubercle bacillus and BCG are known in addition to genetic analyses as methods for identification of Mycobacterium. Specifically, bacterial strains of Mycobacterium can be alkaline-hydrolyzed, and extracted with an organic solvent to provide mycolic acid, which is then analyzed by HPLC, thereby enabling identification of genus and species from the elusion pattern of the mycolic acid (J. Clinical Microbiology, 1327-1330, May 1992).

However, any method to exactly determine the amount of mycolic acid in bacteria-CWS has not yet been known.

DISCLOSURE OF THE INVENTION

The problem to be addressed by the present invention is to provide stable and lyophilizable oil-in-water emulsions comprising bacterial cell wall skeleton components as an effective ingredient, lyophilized formulations of the oil-in-water emulsion of the present invention, bacteria-CWS which is a raw material to prepare the formulations of the present invention and pastes comprising bacteria-CWS which are intermediate materials in the production thereof, processes for preparation thereof, and processes for identification of effective ingredients in the formulations of the present invention and of quantitative assay of the strength thereof, processes for identification of genera, species and strains of the bacteria from which the bacteria-CWS used as an effective ingredient is derived, as well as processes for checking that the present formulations and intermediate materials thereof are identical to the standards.

We have extensively examined compositions of bacteria-CWS, oil, surfactants and stabilizers, etc., and processes for preparation thereof in order to obtain an extremely stable oil-in-water emulsion comprising bacteria-CWS as an effective ingredient and a lyophilized formulation thereof. Firstly, we considered that the properties of a paste consisting of cell wall skeleton components and an oil, which is a intermediate material, are important to successfully prepare lyophilized formulations of an emulsion having an excellent re-suspension stability. Thus, we focused on types and compositions of an oil, and ratios of an oil to cell wall skeleton components as indexes of the properties of a paste, and further examined them. As the result, we have found that a factor which has a great influence on properties of the stable oil-in-water emulsion is a viscosity of the paste comprising cell wall skeleton components which comprises bacteria-CWS and an oil. Further, we have unclosed ratios of cell wall skeleton components to an oil, which provide a good viscosity.

Specifically, it is difficult to make bacteria-CWS into an emulsion with a uniform particle size distribution because bacteria-CWS is insoluble in water and in oil. Particularly, we have found that it is impossible to uniformly disperse bacteria-CWS in an oil upon mixing and stirring when the viscosity of an oil is high and when the amount of the oil is low. Upon examining diverse oils, we have found that a paste comprising bacteria-CWS suitable for emulsification can be obtained at a viscosity below a certain level regardless of the types of the oil. Particularly, emulsification using a paste comprising bacteria-CWS with a viscosity of about 0.7 poise or less was found to provide an emulsion with a good particle size distribution.

On the other hand, although an emulsion can be obtained without any problems using a paste comprising bacteria-CWS with high oil content and low viscosity, an emulsion with a preferred particle size distribution can not be reproduced when the lyophilized formulation is resuspended by adding water. As the result of our study, we have found that a paste comprising bacteria-CWS with a viscosity of about 0.2 poise or more retains its stability of resuspension after lyophilization.

Moreover, lyophilized formulations comprising bacteria-CWS as effective ingredients are different in qualities such as stability, and stability of the oil-in-water formulation obtained after resuspension (resuspension stability) depending on the properties of the paste comprising bacteria-CWS, surfactants, and stabilizers.

International Publication No. WO00/3724 cited above describes that, for the known methods using glycine as a stabilizer, about 900 mM to 1,200 mM (ca. 6.9 % to 9.2 %) glycine is required in order to obtain a stable lyophilized formulation. However, because the biological isotonic concentration of glycine is 300 mM (2.3 %), there is a great concern that such a high concentration would place a burden on living bodies.

We have studied intensively and found that lyophilized formulation with a high resuspension stability could be prepared using mannitol as a stabilizer.

We have studied, and have found that 1 to 10 %, preferably 1 to 5 % mannitol as a stabilizer may be used to enhance the stability of lyophilized formulation, and stability of oil-in-water emulsions after resuspension in case of the oil-in-water emulsion of the paste comprising bacteria-CWS of the present invention.

In addition, we also have studied procedures of preparing the paste comprising bacteria-CWS.

International Publication No. WO00/3724 cited above describes that bacteria-CWS and oil are mixed by firstly diluting with an organic solvent, then removing the solvent by distillation.

The present inventors have studied using various organic solvents as a dispersion-aiding solvent, and found that more excellent pastes comprising bacterial cell wall skeleton components can be obtained by use of a non-polar solvent compared with those using only a polar solvent.

The present invention has been accomplished based on the above findings.

That is, the present invention relates to:

[1] a paste comprising bacteria-CWS which consists of a bacteria-CWS and an oil wherein the paste has a viscosity of 0.7 poise or less (25 °C);

[2] the paste comprising bacteria-CWS according to [1] wherein the paste has a viscosity between 0.2 and 0.7 poise (25 °C);

[3] the paste comprising bacteria-CWS according to [1] wherein the paste has a viscosity between 0.28 and 0.55 poise (25 °C);

[4] the paste comprising bacteria-CWS according to any one of [1] to [3], wherein the particle diameter of the bacteria-CWS is between 0.15 μm and 6 μm;

[5] the paste comprising bacteria-CWS according to [4], wherein D10 % of 0.38 μm or more, and D90% of 0.70 μm or less for the particle diameter of the bacteria-CWS;

[6] the paste comprising bacteria-CWS according to any one of [1] to [5], wherein the bacteria-CWS is BCG-CWS;

[7] the paste comprising bacteria-CWS according to any one of [1] to [6], wherein an oil is selected from the group consisting of squalane, squalene, synthelane 4, peanut oil, camellia oil, soybean oil, liquid paraffin, and ethyl oleate or a mixture of one or more oils thereof;

[8] the paste comprising bacteria-CWS according to [7] wherein the oil is a mixture of two types of oils selected from the group consisting of squalane, squalene, soybean oil, liquid paraffin, and ethyl oleate;

[9] the paste comprising bacteria-CWS according to [8] wherein the oil is a mixture of an oil selected from the group consisting of soybean oil, liquid paraffin and ethyl oleate in admixture with squalane;

[10] the paste comprising bacteria-CWS according to [9], wherein the oil is a 1:1 mixture of ethyl oleate and squalane;

[11] the paste comprising bacteria-CWS according to [7] wherein the oil is squalane;

[12] the paste comprising bacteria-CWS according to any one of [1] to [5], wherein the bacteria-CWS is BCG-CWS and wherein the paste comprises 6.6 g to 35.2 g of squalane per about 0.67 g of BCG-CWS;

[13] a process for preparing a paste comprising bacteria-CWS, which comprises the following steps:

(1) a step of mixing the bacteria-CWS and an oil in an organic solvent as used as a dispersion-aiding solvent; and

(2) a step of removing the organic solvent in (1) by distillation;

[14] the process for preparation according to [13] wherein the organic solvent is an ether solvent;

[15] the process for preparation according to [13] wherein the organic solvent is a hydrocarbon solvent or a halogenated hydrocarbon solvent;

[16] the process for preparation according to [15], wherein the organic solvent is a halogenated hydrocarbon solvent selected from the group consisting of 1,2-dichloroethane, chloroform, and dichloroethane;

[17] the process for preparation according to [15], wherein the organic solvent is a hydrocarbon solvent selected from the group consisting of heptane and hexane;

[18] the process for preparation according to any one of [15] to [17], wherein the organic solvent comprises 5 to 20 % (v/v) of an alcohol solvent;

[19] a paste comprising bacteria-CWS obtainable by the process for preparation according to any one of [13] to [18];

[20] the paste according to [19] wherein the bacteria is BCG bacteria;

[21] the paste according to [19] or [20], wherein the oil is squalane or liquid paraffin;

[22] an oil-in-water emulsion which comprises the paste comprising bacteria-CWS according to any one of [1] to [12], and [19] to [21], a surfactant, a stabilizer, and water;

[23] the oil-in-water emulsion according to [22], which comprises 0.66 g to 3.35 g of the bacteria-CWS, and 0.4 wt% to 8 wt% of the oil per 2L of water;

[24] the oil-in-water emulsion according to [22] or [23], which contains 1 to 10 % mannitol as a stabilizer:

[25] the oil-in-water emulsion according to any one of [22] to [24], which comprises 0.01 % to 3% polyethyleneoxysorbitan fatty acid ester as a surfactant;

[26] the oil-in-water emulsion according to [25], wherein the polyethyleneoxysorbitan fatty acid ester is Tween 80;

[27] the oil-in-water emulsion according to any one of [22] to [26], having the following properties:

(1) the particle diameter of an oil droplet of the emulsion is 0.2 to 30 $\mu$m;

(2) the bacteria-CWS is encapsulated in the oil droplet, and is negative for reaction with lectin;

[28] the oil-in-water emulsion according to [27] wherein D10% is 0.5 $\mu$m or more, and D90% is 20 $\mu$m or less as for the diameter of the oil droplet of the emulsion;

[29] a process for preparation of the oil-in-water emulsion according to any one of [22] to [28], which comprises the following steps:

(1) a step of emulsifying a mixture comprising the paste comprising bacteria-CWS according to any one of [1] to [12] and [19] to [21], and an aqueous solution containing a surfactant at a temperature higher than the turbidity point; and

(2) a step of adding an aqueous solution containing a stabilizer for dilution;

[30] the process for preparation according to [29], wherein step (2) is conducted at a temperature lower than the turbidity point;

[31] the process for preparation according to [29] or [30] wherein the emulsification step in step (1) comprises the following steps:

(3) a step of emulsifying a mixture comprising the paste comprising bacteria-CWS according to any one of [1] to [12] and [19] to [21], and an aqueous solution containing 0.02 % to 0.8 % of a surfactant (rough emulsification step); and

(4) a step of adding an aqueous solution containing a surfactant to the mixture of (3) to adjust the concentration of the surfactant, and vigorously stirring the mixture (complete emulsification);

[32] the process for preparation according to [31], wherein the surfactant is polyethylene oxysorbitan fatty acid ester;

[33] the process for preparation of the oil-in-water emulsion according to [32], wherein the polyoxyethylene oxysorbitan fatty acid ester is Tween 80;

[34] the process for preparation of the oil-in-water emulsion according to any one of [29] to [33], wherein the stabilizer is mannitol;

[35] a lyophilized formulation obtainable by lyophilizing the emulsion according to any one of [22] to [28];

[36] an assembly of bacterial cell wall skeleton component particles (an assembly of bacteria-CWS particles) wherein the particle diameter is 0.15 to 6 $\mu$m in the particle size distribution;

[37] an assembly of bacteria-CWS particles wherein the particle diameter is 0.2 to 2 $\mu$m in the particle size distribution;

[38] the assembly of bacteria-CWS particles according to [36] to [37], wherein the particle size distribution shows a single peak, as well as D10 % of 0.2 or more and D90 % of 0.7 or less;

[39] the assembly of bacteria-CWS particles according to the any one of [36] to [38], wherein the particle size distribution shows a single peak, as well as D10%: $0.23 \pm 0.05$ and D90%: $0.60 \pm 0.05$;

[40] a process for preparation of the assembly of bacteria-CWS particles according to any one of [36] to [39], which comprises dispersing the bacteria-CWS in a solvent containing an aliphatic hydrocarbon solvent;

[41] the process according to [40], wherein the solvent is a mixture of an aliphatic hydrocarbon solvent and an alcohol solvent;

[42] the process for preparation according to [41], wherein the solvent is a heptane containing 5 to 20 % ethanol; and

[42-2] a bacteria-CWS obtainable by the process according to any one of [40] to [42].

Further, in this embodiment, the present invention relates to:

[42-3] the paste comprising bacteria-CWS according to the present invention which comprises an assembly of bacteria-CWS particles, wherein the particle diameter is from 0.1 $\mu$m to 20 $\mu$m, preferably from 0.15 to 6$\mu$m, and more preferably 0.2 $\mu$m to 2 $\mu$m;

[42-4] the paste comprising bacteria-CWS according to [42-3], wherein the assembly of bacteria-CWS particles exhibit a particle size distribution showing a single peak as well as D10%: $0.23 \pm 0.05$ and D90%: $0.60 \pm 0.05$;

[42-5] an oil-in-water emulsion which comprises the paste comprising bacteria-CWS according to [42-3] or [42-4], a surfactant, a stabilizer, and water;

[42-6] a lyophilized formulation obtainable by lyophilizing the emulsion according to [42-5]; and

[42-7] a pharmaceutical composition which consists of the emulsion of the present invention.

Further, methods to identify the genera, species and so on of Mycobacterium bacteria on the basis of the pattern of the long-chain fatty acids which are comprised in CWS of Mycobacterium in an amount of about 30 to 45 % as a constituent, have been known. However, neither method to exactly determine the amount of the long-chain fatty acids comprised in the bacteria-CWS of Mycobacterium, Nocardia and the like, nor methods to identify the genera, species or strains of the bacteria on the basis of the fatty acid from which the bacteria-CWS is derived, has been known yet. We, the present inventors, have studied intensively, and as the results, we have found that the species and strains of bacteria such as Mycobacterium and Nocarida can be identified by extracting and analyzing the long-chain fatty acids contained in the bacteria-CWS, thus enabling the assay of the strength of said bacteria-CWS. Moreover, we have established the method to effectively determine the long-chain fatty acids by converting the acid to the derivative thereof in order to enhance detection sensitivity, and then analyzing by use of HPLC and the like.

On the other hand, the bacteria-CWS of Mycobacterium, Nocardia and the like is a biopolymer consisting of a long-chain fatty acid such as mycolic acid, a saccharide, a peptide glycan and the like, and those components, the ratio of the components and the molecular weight thereof which affect the strength of CWS may vary depending on the culture conditions of the bacteria. Accordingly, homogeneity and strength of the bacteria-CWS of Mycobacterium, Nocardia and the like should be evaluated per each production lots.

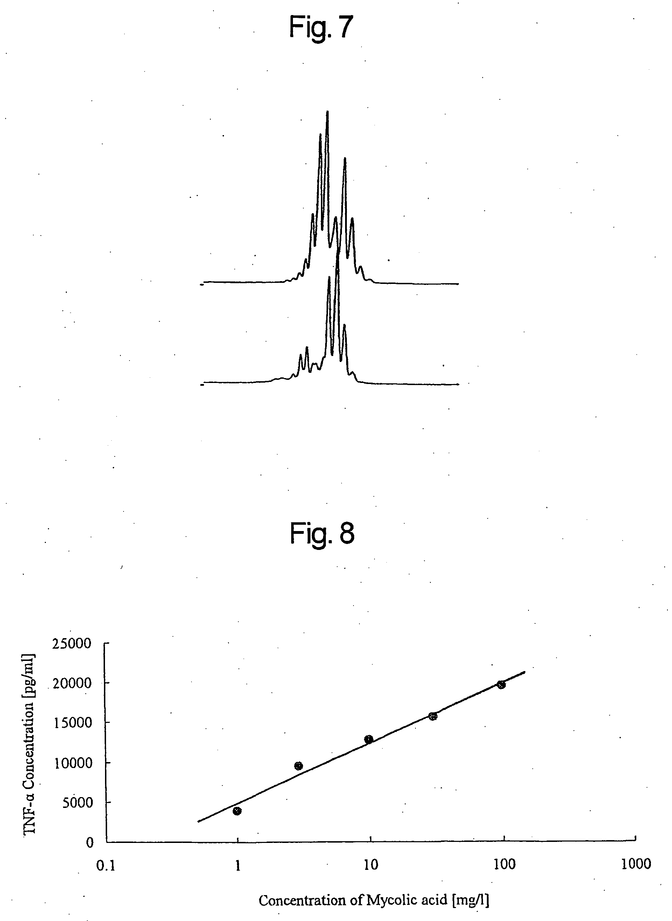

As the results of our intensive study, we have found that the amount of mycolic acid contained in CWS correlates with the biological activity of CWS of BCG (BCG-CWS), and thus established a simple method for assay of strength. Particularly, in BCG-CWS standard, the content of mycolic acid exhibits proportional relationship with bioactivity of BCG-CWS, i.e., TNF-$\alpha$ production induction activity, which is one of immunopotentiating activities (see Fig.8). That is, we have found that the strength of the bacteria-CWS of Mycobacterium, Nocardia and the like as an immunopotentiating agent and/or as anti-tumor agent can be assayed by determining the long-chain fatty acid which is a constituent of bacteria-CWS of Mycobacterium, Nocardia and the like. Further, we have found that the genera, species and strains of the bacteria can be also identified from the chromatograph of the long-chain fatty acid obtained from the strains, raw materials and formulations.

The present invention has been accomplished based on the above findings. Specifically, the present invention relates to:

[43] a process for identification of species and strains of a bacterium from which bacterial cell wall skeleton components (hereinafter referred to as CWS) are derived, which comprises the following steps:

(1) a step of separating and/or extracting the long-chain fatty acid contained in the bacteria-CWS to prepare a long-chain fatty acid fraction, and if necessary, converting the long-chain fatty acid in the long-chain fatty acid fraction into a derivative thereof;
(2) a step of determining the long-chain fatty acid or a derivative thereof in the long-chain fatty acid fraction of (1) by chromatography; and
(3) a step of evaluating for species and strains of a bacterium from which the bacteria-CWS is derived based on the results of determination (2);

[44] the process according to [43] wherein step (2) comprises a step of labeling the long-chain fatty acid in the long-chain fatty acid fraction to prepare a labeled long-chain fatty acid derivative;
[45] the process according to [43] or [44], wherein step (3) in [43] comprises a comparison with a standard of bacteria-CWS;
[46] a process for assay of the strength of bacterial cell wall skeleton components (hereinafter referred to as CWS), which comprises the following steps:

(1) a step of separating and/or extracting the long-chain fatty acid contained in the bacteria-CWS to prepare a long-chain fatty acid fraction, and if necessary, converting the long-chain fatty acid in the long-chain fatty acid fraction into a derivative thereof;
(4) a step of determining the content of the long-chain fatty acid or a derivative thereof in the long-chain fatty acid fraction; and
(5) a step of evaluating for an immunopotentiating activity of the bacteria-CWS based on the results of determination (4);

[47] the process according to [46], wherein step (4) determining the content of the long-chain fatty acid or a derivative thereof comprises a step of labeling fluorescently the long-chain fatty acid in the long-chain fatty acid fraction to prepare a fluorescent-labeled long-chain fatty acid derivative;
[48] the process according to [46] or [47], wherein step (5) evaluating for an immunopotentiating activity of the bacteria-CWS comprises comparing with a standard of bacteria-CWS;
[49] the process according to any one of [43] to [48], wherein the long-chain fatty acid derivative is a long-chain fatty acid ester;
[50] the process according to [49], wherein the long-chain fatty acid derivative is a fluorescent-labeled derivative;
[51] the process according to any one of [43] to [50], wherein the bacteria are those of Mycobacterium or Nocardia;
[52] the process according to [51], wherein the bacteria of Mycobacterium are those of BCG;
[53] the process according to any one of [43] to [52], wherein the long-chain fatty acid is mycolic acid.

BRIEF DESCRIPTION OF THE DRAWING

Figure 1 shows the particle size distribution of BCG-CWS in the paste using heptane as a dispersion-aiding solvent.
Figure 2 shows the particle size distribution of the paste using 90 % heptane/ 10 % ethanol as a dispersion-aiding solvent.
Figure 3 shows the particle size distribution regarding of the oil-in-water emulsion in the squalane 1.6 % formulation before lyophilization.
Figure 4 shows the particle size distribution regarding of the oil-in-water emulsion in the squalane 1.6 % formulation after lyophilization
Figure 5 shows the chromatogram of the test sample, plotting the retention time of HPLC as the horizontal axis and the strength of the fluorescence as the longitudinal axis.

Figure 6 shows a standard carve of BCG-CWS raw material standard.
Figure 7 shows that the strains may be identified from the chromatogram from the difference between strains (top; BCG Tokyo strain, bottom: BCG Pasteur strain).
Figure 8 shows the correlation between the mycolic acid content and biological activity of the test substances.

BEST MODE FOR CARRYING OUT THE INVENTION

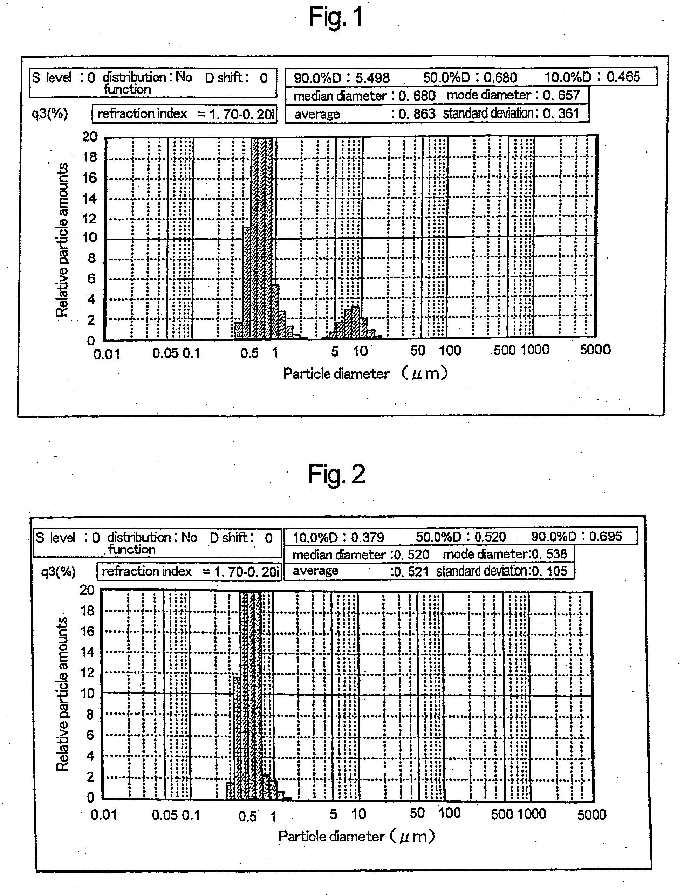

In the present invention, bacteria from which a bacteria-CWS is derived are exemplified by gram-positive rod such as *Mycobacteria, Nocardia, Corynebacteria (Proprionibacteria),* and Rhodococcus, Bordetella, Listeria, Gordona or the like. Preferably, Mycobacteria and Nocardia are included. "Mycobacteria" represents bacteria belonging to acid-fast bacteria, Mycobacterium, particularly includes *Mycobacterium tuberclosis, Mycobacterium bovis* (including BCG), *Mycobacterium africanum, Mycobacterium microti*, as well as *Mycobacterium leprae*, non-tuberclosis acid-fast bacteria, *Mycobacterium kansasii, Mycobacterium avium, Mycobacterium phlei* and the like. Nocarida includes *Nocardia rubra* and the like. Bacteria-CWS may be obtained in form of an insoluble residue by a purification process which comprises crashing the cells with a physical means, removing nucleic acids and proteins from the cell debris, and then delipidating the resultant material, and the preparation thereof is well-known (J. Nat. Cancer Inst., 52, 95-101 (1974)).
The bacterial cell wall skeleton components are used as an oil-in-water emulsion in a concentration of 0.01 to 10 mg/ml. Preferably, the concentration is from 0.1 mg/ml to 2 mg/ml, more preferably from 0.2 mg/ml to 1 mg/ml.
In the present invention, an "oil" includes a mineral oil, or an animal and vegetable oil such as those described in Immunology, 27, 311-329 (1974). Mineral oil is exemplified by a liquid paraffin (Drakeol-6VR, Morescobiores U-6, Moleskobiores U-8, etc.), a bayol (Bayol F), and the like. Vegetable oil is exemplified by a soybean oil, synthelane 4, ethyl oleate, a peanut oil, a camellia oil, a sesame oil, AD-65 (a mixture of a peanut oil, Arlacel, and aluminum monostearate), and the like. Animal oil is exemplified by squalane, and a terpenoid derivative such as squalene, and the like. A mixture of one or more oils selected from animal oils, vegetable oils, mineral oils may be included. Among them, squalane, or a mixture of squalene with a vegetable oil such as a soybean oil, ethyl oleate, oleic acid, etc (or oils derived therefrom), for example, Drakeol-6VR, or a mixture of squalene with a mineral oil such as various liquid paraffin is preferred.
More preferably, squalane, Drakeol-6VR, a mixture of squalane and soybean oil, a mixture of squalane and ethyl oleate, or a mixture of squalane and Drakeol-6VR, and the like are included.
"Viscosity" as used herein means a value determined by dynamic viscoelasticity measurement, for example, values obtained using a co-axial bicylindrical viscometer. In the present invention, viscosity may be measured using a co-axial bicylindrical viscometer manufactured by Rheology Co., Ltd (Model MR-300, Soliquid meter) at 25 °C under nitrogen atmosphere. One poise represents 0.1 Pa.s (pascal.sec).
"An organic solvent as a dispersion-aiding solvent" usable for the preparation of the paste of the present invention includes an organic solvent that can be evaporated off by, for example, heating under a nitrogen stream or under reduced pressure, and may be a mixture of two or more solvents. Particularly, such solvents include those having a boiling point from 30 °C to 140 °C at atmospheric pressure.
When an alcohol solvent such as ethanol is used alone for the preparation of the paste of the present invention, bacteria-CWS may not be sufficiently dispersed. On the other hand, when a non-polar solvent, for example, an aromatic hydrocarbon solvent such as toluene, an aliphatic hydrocarbon solvents such as hexane, heptane and the like, a halogenated hydrocarbon solvent such as chloroform, or ether solvents such as tetrahydrofuran is used, a homogeneously dispersed solution can be obtained, and a paste with an excellent homogeneity can be obtained, those of which have been found by the present inventors. Further, the inventors have found that using a non-polar solvent partially containing an alcohol solvent, a more excellent paste comprising bacteria-CWS can be obtained compared with the case using a non-polar solvent alone. Specifically, using a non-polar solvent alone, the resultant paste contains a small amount of giant particles having a particle diameter of about 10 μm mixed therein, exhibiting two peaks of the particle size distribution of bacteria-CWS in the paste (see Fig.1). When an oil-in-water emulsion is prepared, the formulations of different lots tend to provide variation in particle size distribution. It has been found that when an alcohol solvent is mixed therein, a homogeneous particle size distribution can be obtained.
Preferred dispersion-aiding solvents for the preparation of the pastes according to the present invention include a non-polar solvent, and a mixture of a non-polar solvent and an alcohol solvent. Such organic solvents are preferably selected from those described as a solvent of Class 2 or 3 in the ICH Guideline for Residual Solvents.
Non-polar solvents include, for example, an aromatic hydrocarbon solvent such as toluene, an aliphatic hydrocarbon solvent such as cyclopentane, cyclohexane, pentane, hexane, heptane, octane; a halogenated hydrocarbon solvent such as dichloromethane, chloroform, trichloroethylene; and an ether solvent such as tetrahydrofuran. Such a non-polar solvent is not limited to a single type, but several solvents may be mixed and used as needed.
Alcohol solvents include, for example, methanol, ethanol. propanol, isopropanol, or butanol. Particularly, ethanol is preferred.

When the dispersion-aiding solvent is a mixture of an alcohol solvent and a non-polar solvent, 5 to 30 %, preferably 5 to 20 %, more preferably 5 to 15 % of alcohol solvent is contained.

More preferably, solvents include halogenated hydrocarbon solvents such as chloroform, dichloromethane, trichloroethylene; aliphatic hydrocarbon solvents such as hexane, heptane, pentane, octane; aromatic or aliphatic hydrocarbon solvents containing 0 to 20 % ethanol. Particularly, 5 to 20 % ethanol-heptane, 5 to 20 % ethanol-hexane, 5 to 20 % ethanol-toluene, 5 to 20 % ethanol-cyclohexane, 5 to 20 % propanol-heptane, 5 to 20 % isopropanol-heptane, preferably 10 % ethanol may be exemplified.

A "surfactant" which can be used in the present invention is not limited to a particular species as long as it is used in a pharmaceutical formulation. It includes a phospholipid, a nonionic surfactant, and the like. The phospholipid is exemplified by phosphatidyl amine, phosphatidyl ethanol amine, phosphatidyl inositol, phosphatidyl serine, sphingomyelin, lecithin, or the like. A hydrogenated phospholipid may be used. The nonionic surfactant is exemplified by poly(oxyethylene)-polyoxypropylene copolymer, Polyoxyethylene Hydrogenated castor oil, a poly(oxyethylene) castor oil derivative, a poly(oxyethylene) sorbitan fatty acid ester such as poly(oxyethylene) sorbitan monolaurate (Polysorbate 20), poly(oxyethylene) sorbitan monopalmitate (Polysorbate 40), poly(oxyethylene) sorbitan monostearate (Polysorbate 60), or poly(oxyethylene) sorbitan monooleate (Polysorbate 80), and a sorbitan fatty acid ester such as sorbitan monolaurate (Span 20), sorbitan monopalmitate (Span 40), sorbitan monostearate (Span 60), and sorbitan monooleate (Span 80). Preferred surfactant includes egg yolk phosphatidylcholine, egg yolk lecithin, soybean lecithin, and Polysorbate 80, Polysorbate 20, Polyoxyethylene Hydrogenated castor oil 60 (HCO-60), Polyoxyethylene Hydrogenated castor oil 50 (HCO-50), polyoxyethylene (160)polyoxypropylene(30)glycol (pluronic F68) and the like. More preferred surfactant includes Polysorbate 80.

The surfactant is suitably used in an oil-in-water emulsion in a concentration range of 0.01 to 10 %w/w, and preferably, 0.01 to 3 %w/w. Each surfactant may be used solely, or in combination with other several ones, as appropriate.

"Stabilizers" are components used in order to maintain and improve stability of the above emulsion. The stabilizers which can be used in the present invention include a monosaccharide, a sugar alcohol, a polysaccharide, an amino acid, a protein, urea, or an inorganic salt. The monosaccharide and disaccharide includes glucose, fructose, sucrose, lactose, trehalose and the like. The sugar alcohol includes mannitol, sorbitol and the like. More preferred sugar alcohol includes mannitol. The polysaccharide is exemplified as a preferred one by dextran, starch, maltodextrin, cellulose, polyvinylpyrrolidone, sodium alginate, or the like. The amino acid is preferably a neutral amino acid such as alanine, glycine, proline, and more preferably, glycine among the neutral amino acids. The protein is exemplified as a preferred one by albumin, a gelatin, a collagen, and the like. The inorganic salt includes sodium chloride, calcium chloride, sodium sulfate, sodium carbonate and the like.

The stabilizer is preferably monosaccharide, or sugar alcohol, more preferably mannitol.

Such a stabilizer may be used solely, or in combination with other several ones, if necessary.

The concentration of the stabilizer is suitably in a range of 0.1 to 20 %w/w, and preferably, 0.1 to 10 %w/w. The suitable concentration of the stabilizer varies depending on the types of the stabilizer, and may be adequately adjusted depending on the manufacturing scale and the content of the component, if necessary. As for amino acids such as glycine, the suitable concentration is 2.25 % (300 mM) to 1.25 % (1500 mM), preferably about 6.75 % (900 mM). As for mannitol, the concentration in the oil-in-water emulsion is preferably 1 to 10 %, more preferably 1 to 8 %, even more preferably 3 to 6 %. Since mannitol may be used as a stabilizer at the concentration almost equal to isotonic, stable formulations providing less burden on a living body may be prepared by use of mannitol.

Aqueous solvents as used in resuspension of the lyophilized formulations of the present invention are media for dispersion of emulsion particles, and include distilled water for injection, physiological saline or the like, not being limited to a particular species as long as it can be used as an aqueous solvent for injection.

"Long-chain fatty acids" represent a long-chain fatty acid characteristically existing in cell walls of *Mycobacteria, Nocardia, Corynebacteria, Rhodococcus, Gordona,* or the like. Such a long-chain fatty acid includes, for example, mycolic acid, which represents an $\alpha$-alkyl-$\beta$-hydroxyl fatty acid with a total carbon number of about 22 to 90. Among them, the total carbon numbers of mycolic acid is about 60 to 90 in Mycobacterium, about 44 to 60 in Nocardia. Such mycolic acids may be isolated as a mixture of several types of molecules. Alpha chains of mycolic acids consisting of linear hydrocarbons have different length among different genera and species with a wide distribution mainly ranging from C22 to C66. On the other hand, in $\beta$-chains, it is known that there exist functional groups such as $\alpha$-mycolic acid (with a cyclopropyl group and/or a double bond) in any kinds of Mycobacterium, and various sub-mycolic acids (methoxy, ethoxy, keto, epoxy, methyl, etc.) in certain strains. For example, BCG Tokyo strains have $\alpha$-, methoxy- and keto-mycolic acid.

As used herein, the term "strength" represents a strength of biological activity of bacteria-CWS of Mycobacterium, Nocardia and the like, or medical products comprising them, or a relative value compared with the calibrated standards.

The medical products include a suspension of CWS in an aqueous solvent; a paste comprising CWS and an oil such as a mineral oil, squalane or squalene; an emulsion solution obtained by re-suspending the paste; or a lyophilized formulation obtained by lyophilizing said suspension in an aqueous solvent or emulsion solution.

In this context, biological activity means an immunopotentiating activity and/or an anti-tumor activity of CWS of bacteria such as Mycobacterium, Nocardia and the like, and includes any activity in vivo and in vitro well-known in the art. Particularly, interferon-$\gamma$ induction activity, TNF-$\alpha$ induction activity, anti-tumor activity in the test tumor animal model and the like are included.

As the first aspect, the present invention provides an oil-in-water emulsion, and a paste comprising bacteria-CWS as an effective ingredient, which may be a suitable intermediate material in the production of a lyophilized formulation thereof. Specifically, the present invention provides a paste consisting of a bacteria-CWS and an oil (an oily mixture), which is characterized by having a viscosity of about 0.7 poise or less (25 °C), preferably about 0.2 to about 0.6 poise (25 °C). More preferably, a paste comprising bacteria-CWS with a viscosity from about 0.28 to 0.55 poise (25 °C).

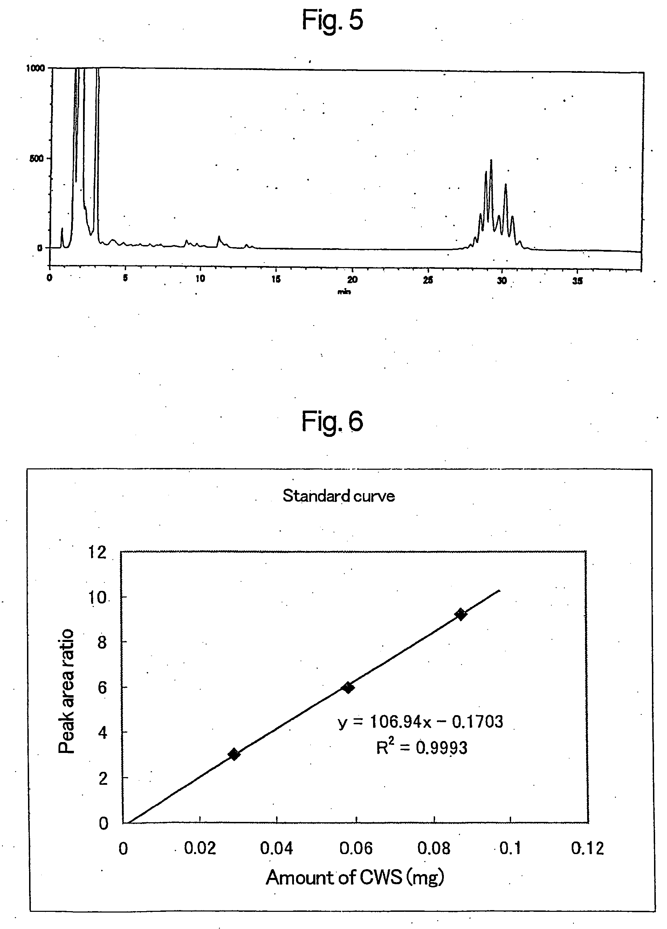

When squalane is used as an oil, a paste comprising bacteria-CWS preferably has a viscosity from about 0.35 to 0.55 poise (25 °C), and more preferably, from about 0.39 to 0.51 poise (25 °C). For example, when an oil-in-water emulsion is prepared from a paste comprising bacteria-CWS (viscosity: 0.43 poise) using 26.7 g squalane per lg of BCG-CWS and lyophilized in the same manner as described below, oil droplets in the oil-in-water emulsion obtained by re-suspending the lyophilized formulation exhibits a single peak with narrow half bandwidth, with particle size being from 2 to 3 $\mu$m (see Fig.4). On the other hand, as for the particle size distribution of the oil droplets in the oil-in-water emulsion obtained by re-suspending the lyophilized formulation, particles with diameter of 10 to 50 $\mu$m may increase in addition to the main distribution with particle diameter of 2 to 3 $\mu$m as the viscosity of the paste comprising BCG-CWS increased, resulting in impaired homogeneity in particle size. Further, as for long-term storage stability of said lyophilized formulation, the paste comprising BCG-CWS with the above viscosity of 0.43 poise is more excellent in terms of the generation of adhesion to the wall of the vessel and the concentration of the raw material in the re-suspended oil-in-water emulsion. The specific embodiment of the paste comprising BCG-CWS is exemplified as a preferred composition by those having a viscosity of about $0.43 \pm 0.3$ poise.

Preferred pastes comprising bacteria-CWS are those comprising an assembly of bacteria-CWS particles wherein the particle diameter is 0.1 $\mu$m to 20 $\mu$m, preferably 0.15 to 6 $\mu$m, more preferably 0.2 $\mu$m to 2 $\mu$m in the particle size distribution, and those wherein the particle size distribution shows a single peak, and D10 % of $0.23 \pm 0.05$ and D90 % of $0.60 \pm 0.05$.

When a mixture with several oils is used, each oils may be mixed in any appropriate composition ratio, but the composition ratio is desirably selected such that the viscosity during mixing with bacteria-CWS is adjusted to about 0.7 poise or less (25 °C). Further, in order to prepare a stable lyophilized formulation after suspension, the composition ratio of each oil may be such that the viscosity during mixing with bacteria-CWS is about 0.2 poise or more (25 °C). Particularly, about 6.6 g to 35.2 g, preferably about 8.4 g to 35. 2 g of squalane may be used for about 0.66 g of bacteria-CWS.

The paste may be prepared in a large scale by: (1) a step of mixing with stirring a bacteria-CWS, an oil, and an organic solvent; and (2) a step of evaporating the organic solvent in step (1) to obtain a paste comprising bacteria-CWS.

The amount of the organic solvent used in step (1) is, for example, 50 ml to 500 ml for 0.67 g of bacteria-CWS. The order of addition of the materials may not be particularly limited. The time for mixing these materials with stirring may not be particularly limited, but preferably from 10 minutes to 1 hour.

In step (2), a heating temperature to evaporate the solvent may be properly selected depending on the boiling point and vapor pressure of the solvent used. Since deactivation of the cell wall skeleton may be occurred at evaluated temperature, the temperature not higher than 100 °C is preferred at which deactivation may not occur. Preferably, the temperature is not higher than 80 °C.

The step of evaporating off the solvent may be carried out under ambient pressure or under reduced pressure.

Further, the paste comprising bacteria-CWS has an assembly of bacteria-CWS particles having a particle diameter of 0.1 $\mu$m to 20 $\mu$m, preferably 0.15 to 6 $\mu$m, still more preferably 0.2 $\mu$m to 2 $\mu$m. That is, pastes comprising bacteria-CWS wherein (1) the viscosity is about 0.2 to 0.7 poise (25 °C), and (2) the particle diameter of the bacteria-CWS particle assembly is 0.15 $\mu$m to 6 $\mu$m, are also a preferable embodiment of the present invention.

As the second aspect, the present invention provides an oil-in-water emulsion which has a homogeneous particle size distribution and which comprises a bacteria-CWS as an effective ingredient, and a pharmaceutical formulation consisting of the oil-in-water emulsion. Specifically, the invention provides an oil-in-water emulsion which comprises the paste comprising bacteria-CWS as described above, 0.1 % to 20 %, preferably 1 % to 10 % of a stabilizer; and 0.01% to 10 %, preferably 0.01 % to 3 % of a surfactant, as well as a pharmaceutical composition thereof.

Specific example includes an oil-in-water emulsion which comprises 0.67 g to 3.35 g of bacteria-CWS and 0.1 to 10 %w/w, preferably 0.4 to 8 %w/w, more preferably 0.6 to 5 % of squalane, as well as 1 % to 10 %w/w of stabilizer, 0.01 % to 3 %w/w of surfactant.

The process for preparation of the oil-in-water emulsion of the present invention is not particularly limited, and those described in International Publication No. WO00/3724 and the like may be used. For example, example of such a process includes a process wherein the paste comprising bacteria-CWS prepared according to the above process is emulsified using a two-step emulsification, that is, emulsification methods 1 and 2 as shown below.

Emulsification method 1:

The oil-in-water emulsion may be prepared by the following steps:

1) a step wherein an aqueous solution containing a surfactant at a low concentration (about 10 % or less of the oil concentration) is added to the cell wall skeleton components oily mixture (paste), and then the resultant mixture is stirred gently to perform a rough emulsification;
2) a step wherein the concentration of the roughly emulsified emulsion solution of step 1) is adjusted by adding a surfactant and a stabilizer in amount to provide the desired final concentration, and the mixture is stirred vigorously with a dispersing/emulsifying device at a temperature from room temperature to 100 °C to conduct a complete emulsification.

Dispersing/emulsifying devices usable in the present invention include a Potter-Elvehjem type homogenizer, a homomixer, an ultrasonication homomixer, Microfluidizer (trade name), Nanomizer (trade name), Ultimizer (trade name), a Manton-Gaulin homogenizer type high pressure homogenizer, all of which can be used to conduct dispersion or emulsification to prepare a desired oil-in-water emulsion. For some reason in the preparation, additives such as an excipient or a stabilizer may be added to the resultant oil-in-water emulsion.

Emulsification method 2

The oil-in-water emulsion of the present invention may be prepared by the following steps:

4) a step wherein an aqueous solution containing a surfactant at a low concentration (about 10 % or less of the oil) is added to the cell wall skeleton components oily mixture (paste), and then the mixture is gently stirred to conduct a rough emulsification;
5) a step wherein the concentration of the roughly emulsified emulsion solution of step 4) is adjusted by adding a surfactant to bring the final concentration to about 0.1 % to 3 %, then a complete emulsification is conducted at a temperature from room temperature to 100 °C by vigorously stirring with a dispersing/emulsifying device;
6) a step wherein the emulsion solution of step 5) is cooled to a temperature lower than the turbidity point if necessary when the stabilizer used is an electrolyte such as an amino acid or an inorganic salt, then an aqueous solution containing a surfactant to bring the final concentration to 0.1 % to 3% and a stabilizer to bring the final concentration to 1.0 % to 10 % is added for dilution. Preferably, in step 6), an oil-in-water emulsion may be prepared by diluting the emulsion obtained in step 5) to 2 to 10 times.

The preparation of the oil-in-water emulsion of the present invention using emulsification method 2 as described above avoids the precipitation of a surfactant, which would be a problem encountered when an emulsion containing en electrolyte such as an amino acid, an inorganic salt or the like as a stabilizer in the preparation using emulsification method 1. Moreover, dilution step 6) in emulsification method 2 does not change the particle size distribution of the emulsion. A large scale production using emulsification method 1 requires using an emulsifier device suitable depending on preparation scale, which limits the preparation scale. However, using a high-concentration emulsion solution as an intermediate material in emulsification method 2 provides an advantage to increase the amount of oil-in-water emulsion prepared per batch.

In the step of preparation of the oil-in-water emulsion in emulsification method 1 or 2 as described above, an agent for rendering isotonic may be added if necessary. These agents for rendering isotonic are not limited to a single type, but may be used in a combination of several types as appropriate. In the present invention, an agent for rendering isotonic may also function as an excipient that forms a lyophilized preparation into a lyophilized cake. An agent for rendering isotonic or an excipient is exemplified by a saccharide, an amino acid, an urea, an inorganic salt, and the like. The saccharide is exemplified by a monosaccharide, a disaccharide, and a sugar alcohol. The monosaccharide includes glucose, fructose, and the like, the disaccharide includes maltose, lactose, trehalose, sucrose, and the like, and the sugar alcohol includes mannitol, sorbitol, and the like. The amino acid includes alanine, glycine, lysine, arginine, proline and the like. Each agent for rendering isotonic may be used solely, or in combination with other several ones, if necessary. As an isotonic agent, those described for the stabilizer may be used. A surfactant may also serve as an isotonic agent. The isotonic agent may be different from the stabilizer. The concentration of the isotonic agent may be selected depending on the contents of the other components, and suitably used in a concentration range of 0.1 to 30 %w/w in the oil-in-water emulsion.

The oil-in-water emulsion of the present invention has oil droplets with particle diameter of 0.2 to 30 μm, preferably 0.2 to 20 μm, of which the average particle diameter is preferably 2 to 3 μm.

Bacteria-CWS is encapsulated in an oil droplet.

Whether or not bacteria-CWS is encapsulated in an oil in the oil-in-water emulsion of the present invention may be examined by assay of the agglutination reaction of the cell wall skeleton components with lectin. As such assay, methods, for example, described in International Publication No. WO00/3724 may be used.

Thus, an oil-in-water emulsion negative for agglutination reaction with lectin, wherein (1) the particle diameter of the emulsion oil droplet is 0.2 to 10 μm, with the average particle diameter being 2 to 3 μm; and (2) bacteria-CWS is encapsulated in the droplet, may be also encompassed as an embodiment of the present invention. The particle diameter of the oil droplet of the emulsion may be preferably D10%: 0.5 μm or more, and D90 %: 20μm or less.

As the third aspect, the present invention provides a lyophilized formulation obtainable by lyophilizing the above oil-in-water emulsion.

The lyophilized formulation may be prepared by lyophilizing the above oil-in-water emulsion. That is, the lyophilized formulation of the present invention is obtained by subjecting the oil-in-water emulsion to lyophilization process, and finally substituting the content of the vial with nitrogen and sealing the vial.

For lyophilization of the oil-in-water emulsion, the temperature and time for such lyophilization may not be particularly limited, and include, for example, those described in International Publication No. WO00/3724.

As an aqueous solvent used for preparation of the oil-in-water emulsion by resuspending the lyophilized formulation, the isotonic solutions described above may be used. The lyophilized formulation can be more quickly resuspended upon addition of a suitable aqueous solvent, thus providing an oil-in-water emulsion showing a particle size distribution and stability similar to those before the lyophilization.

As the fourth aspect, the present invention provides an assembly of bacterial cell wall skeleton component particles (a bacteria-CWS particle assembly) which has a particle diameter of 0.15 to 6 μm, preferably 0.2 to 2 μm in the particle size distribution. The invention also comprises an assembly of bacteria-CWS particles in which the particle diameter shows D10%: 0.2 μm or more, and D90%: 0.7 μm or less, wherein "D 10 %: 0.2 μm" means that its particle diameter reaches 10 % of the assembly summation as calculated in order of increasing diameter in the particle size distribution, and in other words, means that up to 90 % of the bacteria-CWS particle assembly may have a particle diameter of 0.2 μm or more, and wherein "D90%: 0.7 μm" means that its particle diameter reaches 90 % of the assembly summation as calculated in order of decreasing diameter in the particle size distribution, and in other words, means that up to 90 % of the bacteria-CWS particle assembly may have a particle diameter of 0.7 μm or less, preferably an assembly showing D10%: 0.23 ± 0.05 μm, and D90%: 0.6 ± 0.05 μm.

The present invention also encompasses a paste comprising the assembly of bacterial cell wall skeleton component particles (a bacteria-CWS particle assembly) with the particle diameter described above, an emulsion and a lyophilized formulation prepared from the paste. Specifically, an emulsion comprising the above bacteria-CWS (oil droplet) having a particle diameter of 0.2 to 30 μm, preferably 0.3 to 20 μm, more preferably 1 to 10 μm, and a lyophilized formulation thereof may be encompassed in the present invention.

Particle diameter as used herein may be determined by measuring a particle size distribution. Such particle size distribution may be measured according to the methods well-known in the art, for example, using a laser diffraction particle size analyzer (SALD3000, SHIMADZU Corp.) or a MICRO-TURRAX UPA (manufactured by HANEWEL) for a suspension of bacteria-CWS in a solvent. In case of bacteria-CWS having a particle diameter of 1 μm or less, the particle diameter may be preferably measured using MICRO-TURRAX UPA. For example, BCG-CWS suspended in a solvent at a concentration of about 0.1 mg/mL may be used as a sample for measurement.

In case of a paste comprising bacteria-CWS or an emulsion, it may be measured using a laser diffraction particle size analyzer (SALD3000, SHIMADZU Corp.). For example, a paste comprising bacteria-CWS of the present invention may be diluted about 300 times or more with an oil to provide a sample for measurement. Preferably, a sample for measurement may be prepared to adjust the bacteria-CWS concentration to 0.1 to 0.2 mg/mL.

The oil-in-water emulsion of the present invention can be administered parenterally, for example, by injection. Dosage form to be administered may be varied depending on a therapeutic purpose, and is not limited to a particular form. Dosage form usually used includes an injection form for transcutaneous administration. The amount of bacteria-CWS in the emulsion is generally in a range from 0.2 mg/ml to 0.6 mg/ml. The lyophilized formulation of the present invention may be resuspended in an appropriate amount of an aqueous solvent such as the above isotonic solution before use. When the lyophilized formulation is resuspended to administer to a living body, the concentrations of the individual components may be different to those in the oil-in-water emulsion as a production intermediate material before lyophilization. The appropriate amount of the aqueous solvent may be preferably in a range from 0.5 to 4 times of the amount of the solution before lyophilization.

Although the amount and the number of dosage to be administered may be varied depending on the disease to be treated, the symptom, the age, the body weight, sex and the like of a particular patient, it is possible to usually administer 10 to 250 µg, and preferably 25 to 200 µg per administration every week or every four weeks to an adult in case of a parenteral administration, particularly injection.

As the fifth aspect, the present invention provides a process for qualitative identification and determination of the species and strains of Mycobacterium, Nocardia and the like on the basis of chromatogram of the long-chain fatty acid contained in their bacteria-CWS, which comprise the following steps:

(1) a step of separating and/or extracting the long-chain fatty acid contained in the bacteria-CWS to prepare a long-chain fatty acid fraction, and if necessary, converting the long-chain fatty acid in the long-chain fatty acid fraction into a derivative thereof;
(2) a step of determining the long-chain fatty acid or a derivative thereof in the long-chain fatty acid fraction of (1) by chromatography; and
(3) a step of identifying species and strains of a bacterium from which the bacteria-CWS is derived based on the results of determination (2).

Each step will be described in detail.

(1) A step of separating and/or extracting the long-chain fatty acid contained in the bacteria-CWS to prepare a long-chain fatty acid fraction, and if necessary, converting the long-chain fatty acid in the long-chain fatty acid fraction into a derivative thereof;

A defined amount of bacteria-CWS of Mycobacterium, Nocardia and the like is weighed as a sample and the sample is reacted with a base in a solvent within a temperature range from 10 °C to the boiling point of the solvent to conduct hydrolysis. The solvent used may not be particularly limited and any organic solvent may be used so long as it contains sufficient amount of water to conduct hydrolysis. Example of such an organic solvent includes, for example, alcohol solvents such as ethanol, methanol, isopropanol, ethylene glycol; ether solvents such as tetrahydrofuran, diethyl ether, dioxane; hydrophilic solvents such as acetone, dimethylsulfoxide; aromatic hydrocarbon solvents such as toluene, xylene; aliphatic hydrocarbon solvents such as hexane, heptane; halogenated hydrocarbon solvents such as chloroform, dichloromethane, dichloroethane; or a mixture of any of these organic solvents.

The above organic solvent is exemplified preferably by an alcohol solvent such as ethanol; or a mixture of the alcohol solvent and a hydrophobic organic solvent, for example, an ethanol/toluene/water mixture.

The reaction temperature may be properly selected depending on the types of the organic solvent used. Preferably, a method involving heating from room temperature to the boiling point of the solvent, or using an autoclave at 105 °C to 135 °C may be used.

When heated, the reaction may be continued over 5 minutes to 72 hours, preferably over 5 minutes to 5 hours to analyze a pattern of the long-chain fatty acids, and preferably over 30 minutes to 5 hours to quantitatively determine the amount of the long-chain fatty acids. When using an autoclave, the reaction may be continued over 5 minutes to 8 hours. Preferably, the reaction time is from 5 minutes to 3 hours for analyzing the pattern, and from 10 minutes to 5 hours for quantitative determination.

The base used may be any of those well-known in the art, and includes, for example, inorganic salts such as sodium hydroxide, potassium hydroxide, barium hydroxide, sodium carbonate, potassium carbonate, barium carbonate, cesium carbonate.

When a test sample is a bacteria-CWS, it is preferred that a portion of the sample may be weighed per se to improve reaction efficiency, or the sample may be suspended in an organic solvent selected from toluene, chloroform, heptane, ethanol and the like, or a mixture thereof, then a required amount is sampled, and subjected to hydrolysis after evaporation of the organic solvent. When a test sample is a "bacterium" or a "construct component thereof", it may be hydrolyzed while suspended in water similar to the raw material, or, in order to improve reaction efficiency, it may be preferably heated up to the boiling point, then reacted for 5 minutes to 72 hours, or reacted for 5 minutes to 8 hours using an autoclave. Pharmaceutical formulation may be formed into an emulsion upon addition of water, which is entirely or partly taken and subjected to hydrolysis. In addition, in order to improve reaction efficiency, the reaction may be preferably conducted by directly adding a hydrolysis solution rather than by adding water to form an emulsion.

After completion of hydrolysis, an acid is added to bring the reaction solution acidic, and then long-chain fatty acid fraction is extracted with a hydrophobic organic solvent. The acid used may be any one well-known in the art. For example, an inorganic acid such as hydrochloric acid, sulfuric acid, phosphoric acid; an organic acid such as trifluoroacetic acid, acidic ion exchange resin having a sulfone group, and the like may be used. The hydrophobic solvent may not be particularly limited so long as it can be separated from an aqueous layer, and include, for example, hexane, heptane, diethyl ether, toluene, chloroform.

(2) A step of determining the long-chain fatty acid or a derivative thereof in (1) by chromatography;

The chromatogram of the long-chain fatty acid or a derivative thereof contained in the long-chain fatty acid fraction obtained in step (1) is obtained, and the elusion patterns thereof are examined. Elusion patterns mean parameters such as shape of an elusion peak, retention time of each elusion peak, peak area, peak width, or a maximum; and any properties in the chromatogram such as a ratio of the parameters of the elusion peaks. Any method for determination known to those skilled in the art may be used. The long-chain fatty acid of a derivative thereof which is prepared by esterification or amidation of the long-chain fatty acid may be analyzed by HPLC, GC, TLC, etc., providing chromatogram of the long-chain fatty acid or a derivative thereof. The chromatogram of the long-chain fatty acid or labeled derivative thereof means a chromatogram e.g., an elution pattern of peak of HPLC or GC, or a detection pattern of spot in TLC. The method for detection may not be particularly limited, and fluorescence, UV-VIS, radioisotope, RI (differential refractometer), mass spectrometry, electrochemical, chemiluminescence, evaporative light scattering, or laser excitation absorption and the like may be used.

Preferably, the process may include a step wherein the long-chain fatty acid is reacted with a labeling agent to prepare a labeled long-chain fatty acid derivative, and then the eluted pattern thereof is analyzed.

The method to label the long-chain fatty acid include, for example, those wherein the carboxylic acid or hydroxyl group of the long-chain fatty acid is derivatized to a long-chain fatty acid ester or long-chain fatty acid amide which have atoms or partial structure detectable with fluorescence, UV-VIS, radioisotope, RI (differential refractometer), mass spectrometry, electrochemical, chemiluminescence, evaporative light scattering, or laser excitation absorption.

The labeled long-chain fatty acid derivatives include, for example, those having bromomethyl (bromoacetyl), diazomethyl, methyl, hydrazino and the like, which may react with a carboxylic group of the long-chain fatty acid, as well as substituents detectable with ultraviolet, visible or fluorescent radiation, such as 9-anthrylmethyl ester, p-bromophenacyl ester, 6,7-dimethoxy-4-coumarinylmethyl ester, 2-(2,3-naphthalimide)ethyl ester, 2-(2,3-anthracenedicarboxyimide)ethyl ester and the like.

A suitable reaction conditions for the derivatization of particular labeled long-chain fatty acid derivatives may be selected from methods well-known in the art. Generally, a long-chain fatty acid may be reacted with a labeling agent at a temperature from - 10 °C to the boiling point of the solvent for 5 minutes to 72 hours in the presence of an adjuvant such as a base or an acid, if desired, to prepare a labeled long-chain fatty acid derivative. For example, as for 9-anthrylmethyl ester, the long-chain fatty acid fraction may be reacted using a fluorescent labeling agent such as ADAM (9-anthryldiazomethane) at the temperature from - 10 °C to the boiling point of the solvent for 5 minutes to 72 hours, preferably at 20 °C to 60 °C for 3 to 12 hours, to provide a long-chain fatty acid 9-anthranylmethyl ester.

The determination methods as used are those well-known in the art, including HPLC, GC, and TLC. For example, a specific patter of multiple peaks containing a long-chain fatty acid or a labeled long-chain fatty acid derivative eluted may be obtained depending on the properties of a long-chain fatty acid or a labeled long-chain fatty acid derivative to be determined, by HPLC reversed phase chromatography, normal phase chromatography, hydrophobic chromatography, ionexchange chromatography, ion chromatography, size exclusion chromatography, affinity chromatography. Methods for detection include, for example, fluorescence analysis, UV-VIS analysis, radio isotope analysis, RI (differential refractometer) analysis, mass spectrometry, electrochemical analysis, chemiluminescence, evaporative light scattering, or laser excitation absorption and the like may be used corresponding to the above labeling methods.

(3) A step of identifying species and strains of a bacterium from which the bacteria-CWS is derived based on the results of determination (2):

Generally, the species and strains of the test sample, i.e., bacteria-CWS to be examined may be identified and determined by comparing patterns of a chromatogram result of (2) of the test sample with that of the bacteria-CWS standard. Further, it is possible to determine whether or not a bacteria-CWS, which is a sample to be tested, would be identical to the standard sample of the same species and strains in terms of its constituents. That is, although certain culture conditions may provide different constituents among the same species and strains, the sample may be determined to be identical to the standard in terms of constituents of the CWS when comparing the results of analysis (2) with those of the standard.

For example, when the long-chain fatty acid is mycolic acid, a characteristic peak eluted at about 5 to about 120 minutes using a reversed phase HPLC column (a column having an octadecyl group (C 18) or a column having triacontyl group (C30) (2 - 10 mmΦ x 5 - 30 cm, particle diameter, 2 - 10 μm), with gradient conditions using water and/or methanol and 2-propanol, methanol and toluene, or methanol and toluene corresponds to the pattern of respective long-chain fatty acids.

As the sixth aspect, the present invention provides a process for determination and evaluation of a CWS content in a test sample containing bacteria-CWS of Mycobacterium, Nocardia and the like, and to assay and evaluate the amount of the effective ingredient, i.e., strength, which comprises:

(1) a step of separating and/or extracting the long-chain fatty acid contained in the bacteria-CWS to prepare a long-chain fatty acid fraction, and if necessary, converting the long-chain fatty acid in the long-chain fatty acid fraction into a derivative thereof;

(4) a step of determining the content of the long-chain fatty acid or a derivative thereof obtained in step (1);

(5) a step of evaluating for an immunopotentiating activity of the bacteria-CWS based on the results of determination (4).

The each of the above steps will be described in detail.

(1) A step of separating and/or extracting the long-chain fatty acid contained in the bacteria-CWS to prepare a long-chain fatty acid fraction, and if necessary, converting the long-chain fatty acid in the long-chain fatty acid fraction into a derivative thereof

This step is as described above.

(4) a step of determining the content of a long-chain fatty acid or a derivative thereof obtained in step (1)

The fraction comprising the long-chain fatty acid obtained in step (1) may be analyzed by the method described in step (2), and the amount of the long-chain fatty acid and a derivative thereof may be determined from a chromatogram. That is, the long-chain fatty acid may be quantitatively determined by calculating a sum of area of multiple peaks corresponding to a long-chain fatty acid or a derivative thereof in the above chromatogram (2).

In the quantitative determination of the raw material of formulation comprising the bacteria or bacteria-CWS as an effective ingredient, if an internal standard may be used, such standard may not be particularly limited so long as it can be separated and detected from the test sample or a standard by analysis such as HPLC. Such standard may be added during any of steps (1), (2), and (4).

(5) a step of evaluating an immunopotentiating activity of the bacteria-CWS based on the result of determination (4)

Generally, the value of the test sample, that is, of the bacteria-CWS to be tested obtained in (4) may be compared with that of the CWS standard (bacteria of the same species and strains as the test sample) to assay the strength of the test sample. For example, in BCG-CWS Tokyo strains, the long-chain fatty acid is mycolic acid, and the content of the mycolic acid of the test sample obtained in steps (1) and (4) is compared with that of the BCG-CWS Tokyo standard, to assay the strength of the test sample.

The value obtained in (4) of the test sample, i.e., of a bacteria-CWS to be evaluated may be compared with that of the bacteria-CWS standard to assay the strength of the sample.

For example, the value obtained in (4) of the standard of the bacteria-CWS of Mycobacterium, Nocardia and the like (Vstd) may be compared with that of the test sample (Vsam), and the strength of the test sample may be expressed by the following formula:

$$\text{strength (\%)} = V_{sam}/V_{std} \times 100 \ (\%).$$

Preferably, the values of the standard obtained in (4) are measured at different concentrations and several times, and a standard carve is created showing the correlation between the concentration of the standard and the long-chain fatty acid content. The amount of the effective ingredient in the test sample, i.e., strength, can be calculated from the measurement of the long-chain fatty acid content in the test sample using the standard curve.

According to the present processes, not only bacteria-CWS of Mycobacterium, Nocardia, and the like, but also an effective ingredient of a pharmaceutical formulation comprising the bacteria-CWS of Mycobacterium, Nocardia, and the like, as well as a strength may be detected and determined. That is, according to processes similar to those described above, a suspension of strains or pharmaceutical formulations in water or organic solvent is prepared and the amount of the long-chain fatty acid in the strain or the pharmaceutical formulations may be measured.

Examples

The present invention is further illustrated by the examples as described below, but is not restricted by them in any respect.

### Example 1

Preparation of pastes comprising BCG-CWS

BCG-CWS (670 mg), as a cell wall skeleton components obtained as described in J. Nat. Cancer Inst., 52, 95 - 101 (1974), was added to a mixture of squalane (8.4 g) used as an oil and toluene (200 mL), and the resultant mixture was shaken or treated with ultrasonic at room temperature to perform dispersion. Then, the dispersion mixture was heated at 60 - 70 °C under a flow of nitrogen or air to evaporate the toluene. Thus, a paste comprising BCG-CWS was obtained.

The viscosity of the paste was measured at 25 °C using a co-axial bicylindrical viscometer manufactured by Rheology Co., Ltd (Model MR-300, Soliquid meter). The viscosity was measured to be 0.545 poise.

Similarly, using a mixture of squalane as an oil and ethyl oleate (8.4 g), a paste comprising BCG-CWS was obtained. Similarly, the viscosity of the paste was measured at 25 °C using a co-axial bicylindrical viscometer manufactured by Rheology Co., Ltd (Model MR-300, Soliquid meter). The results are shown in Table 1.

Table 1

|  | BCG-CWS | Squalane | Ethyl oleate | Viscosity (poise) |
|---|---|---|---|---|
| 1 | 670 mg | 8.4 g | 0 | 0.545 |
| 2 | 670 mg | 6.3 g | 2.1 g | 0.258 |
| 3 | 670 mg | 4.2 g | 4.2 g | 0.260 |
| 4 | 670 mg | 2.1 g | 6.3 g | 0.165 |
| 5 | 670 mg | 0 | 8.4 g | 0.112 |
| 6 | 0 | 8.4 g | 0 | 0.239 |

As shown in Table 1, the viscosity of squalane was 0.236, and that of a paste obtained by mixing the squalane with BCG-CWS was 0.545. When the content of the ethyl oleate, which has a lower viscosity, was increased, the viscosity of the paste comprising BCG-CWS was reduced accordingly.

### Example 2

Preparation of pastes comprising BCG-CWS

According to a similar procedure to that of Example 1 except that BCG-CWS (670 mg) as a cell wall skeleton, and soybean oil (8.4 g) as an oil were used, an oily mixture (paste) was prepared and measured for its viscosity.

Similarly, a paste was prepared using Synthelane 4, Drakeol as oil, and the viscosity was measured.

The results are shown in Table 2.

Table 2

|  | BCG-CWS | Oil (8.4 g) | Viscosity (poise) |
|---|---|---|---|
| 1 | 670 mg | Soybean oil | 6.95 |
| 2 | 670 mg | Squalane | 0.55 |
| 3 | 670 mg | Synthelane 4 | 0.49 |
| 4 |  | Drakeol | 0.28 |
| 5 | 670 mg | Ethyl oleate | 0.11 |

As shown in Table 2, the viscosity of the pastes comprising BCG-CWS varied considerably depending on the oil used other than squalane.

### Example 3

Changes in the amount of squalane and the viscosity

According to a similar procedure to that of Example 1 except that BCG-CWS (670 mg) was used as a cell wall skeleton, and the amount of squalane varied from 4.2 g to 33.6 g, pastes were prepared and the changes in viscosity were measured.

The results are shown in Table 3.

Table 3

|   | BCG-CWS | Squalane | Viscosity (poise) |
|---|---------|----------|-------------------|
| 1 | 670 mg | 4.2 g | 0.888 |
| 2 | 670 mg | 6.3 g | 0.672 |
| 3 | 670 mg | 8.4 g | 0.545 |
| 4 | 670 mg | 12.6 g | 0.492 |
| 5 | 670 mg | 16.8 g | 0.429 |
| 6 | 670 mg | 33.6 g | 0.362 |

As shown in Table 3, the viscosity tended to increase when the ratio of squalane to BCG-CWS was decreased, and tended to decrease when the ratio of squalane was increased.

Example 4

Changes in the ratio of squalane and the availability of the formulation

To paste 3 of Example 3, 0.02 w/w% Polysorbate 80 aqueous solution (281.5 g) was added, and roughly emulsified using a homomixer, followed by adding 5.2 g of 10 w/w% Polysorbate 80 aqueous solution to conduct a complete emulsification. Finally, 204.2 g of 10 w/w% Polysorbate 80 solution was added and mixed, and then the final concentration of Polysorbate 80 was adjusted to 1.0 w/w% to obtain an oil-in-water emulsion. Subsequently, 1500 mL of 4 w/w% mannitol aqueous solution was added to give 2L of final product, which was an emulsion of BCG-CWS with concentration of 0.3 mg/ml.

Similarly, an emulsion was prepared using the paste in Example 3 (except for 3), and the effect of the amount of the squalane on the availability of the emulsion formulation was examined. The availability of the formulation was evaluated based on: (1) emergency of the assembly during emulsification; (2) particle size distribution immediately after emulsification; (3) particle size distribution before and after lyophilization; and (4) properties after resuspension of the lyophilized formulation.

The results are shown in Table 4. In Table 4, "○" means that the product meets all of the above criteria, "x" means that the formulation fails to meet these criteria, and "Δ" means that the formulation was slightly inferior in terms of homogeneous particle size distribution.

Table 4

|   | BCG-CWS | Squalane | Viscosity (poise) | Availability of formulation |
|---|---------|----------|-------------------|-----------------------------|
| 1 | 670 mg | 4.2 g | 0.888 | X |
| 2 | 670 mg | 6.3 g | 0.672 | Δ |
| 3 | 670 mg | 8.4 g | 0.545 | ○ |
| 4 | 670 mg | 12.6 g | 0.492 | ○ |
| 5 | 670 mg | 16.8 g | 0.429 | ○ |
| 6 | 670 mg | 33.6 g | 0.362 | ○ |

In the preparation of the emulsion using the paste comprising BCG-CWS described in Example 3, as shown in table 4, a good emulsion was prepared using a paste with a viscosity of about 0.7 poise or less. On the other hand, a paste 1 which has higher viscosity (0.888 poise) failed to provide a good emulsion formulation. It indicates that over-emulsification was partially occurred in droplets with the increased viscosity, resulting in emergency of raw material assembly encapsulated with an extremely small amount of oil (substance which is not homogeneously suspended but assembly), which considerably degrades the properties and stability of the formulation.

On the other hand, a paste with viscosity around 0.670 poise did not provide drug assembly, but was poor in homogeneity in the particle size distribution of droplets of the emulsion. The formulation with viscosity of 0.545 poise or less showed a sharp particle size distribution with high homogeneity of droplets of the emulsion (data not shown).

Example 5

Types of oils and the availability of the emulsion formulation

According to a similar procedure to that of Example 4, except that oils used were those in Example 2, an emulsion was prepared and the availability of the formulation was evaluated. The results are shown in Table 5.

Table 5

|  | BCG-CWS | Oil (8.4 g) | Viscosity (poise) | Availability of formulation |
|---|---|---|---|---|
| 1 | 670 mg | Soybean oil | 6.95 | X |
| 2 | 670 mg | Squalane | 0.55 | ○ |
| 3 | 670 mg | Synthelane 4 | 0.49 | ○ |
| 4 | 670 mg | Drakeol | 0.28 | ○ |
| 5 | 670 mg | Ethyl oleate | 0.11 | ○ |

As shown in Table 5, good emulsion was obtained using a paste comprising BCG-CWS having a viscosity of 0.55 poise or less, regardless of the types of the oil. In Table 5, "○" means that (1) the particle size distribution immediately after lyophilization shows a sharp peak with an average particle diameter of 2 to 3 $\mu$m, (2) there is no change in particle size distribution before and after lyophilization, (3) there is no adhesion of the formulation to the vial after resuspension of the lyophilized formulation nor decrease of the concentration of raw material in the formulation. Especially, the formulations (2) and (3) having viscosity of 0.49 - 0.56 poise provided good results.

Example 6

Composition of the oil and the availability of the formulation

According to a similar procedure to that of Example 4, using oil having the same composition as that in Example 1, an emulsion was prepared, and availability of formulation was evaluated. The results are shown in Table 6.

Table 6

|  | BCG-CWS | Squalane | Ethyl oleate | Viscosity (poise) | Availability of formulation |
|---|---|---|---|---|---|
| 1 | 670 mg | 8.4 g | 0 | 0.545 | ○ |
| 2 | 670 mg | 6.3 g | 2.1 g | 0.258 | ○ |
| 3 | 670 mg | 4.2 g | 4.2 g | 0.260 | ○ |
| 4 | 670 mg | 2.1 g | 6.3 g | 0.165 | ○ |
| 5 | 670 mg | 0 | 8.4 g | 0.112 | ○ |
| 6 | 0 mg | 8.4 g | 0 | 0.239 | ○ |

Table 6 shows that the change in the composition of the oil (a mixed ratio of squalane and ethyl oleate) has little effect on the properties of the emulsion, and that the paste comprising BCG-CWS having a viscosity of 0.545 or less provides good emulsion.

Example 7

The change in the amount of squalane and the availability of the formulation

The oil-in-water emulsion prepared according to Example 4 was dispensed into 10 mL aliquots in vials, and then lyophilized to provide the lyophilized formulation of the present invention. Lyophilization was conducted using a lyophilizer (GT-6, manufactured FINTEC). Then the availability of the preparation was evaluated in terms of resuspension stability, particle size distribution, and the like. The results are shown in Table 7.

Table 7

|  | BCG-CWS | Squalane | Viscosity (poise) | Availability of formulation |
|---|---|---|---|---|
| 1 | 670 mg | 4.2 g | 0.888 | X |

Table 7   (continued)

|   | BCG-CWS | Squalane | Viscosity (poise) | Availability of formulation |
|---|---------|----------|-------------------|----------------------------|
| 2 | 670 mg | 6.3 g | 0.672 | Δ |
| 3 | 670 mg | 8.4 g | 0.545 | ○ |
| 4 | 670 mg | 12.6 g | 0.492 | ○ |
| 5 | 670 mg | 16.8 g | 0.429 | ○ |
| 6 | 670 mg | 33.6 g | 0.362 | ○ |

As shown in Table 7, the availability of emulsion was also influenced by the viscosity of the paste comprising BCG-CWS. The paste comprising BCG-CWS having a viscosity of 0.672 or less provides a good lyophilized formulation. That with viscosity of 0.545 or less is found to provide more excellent lyophilized formulation.

Example 8

The composition of the oil and the availability of the formulation

The lyophilized formulation of the oil-in-water emulsion prepared according to Example 4 was prepared, and evaluated for availability of formulation in terms of resuspension stability, particle size distribution and the like. The results are shown in Table 8.

Table 8

|   | BCG-CWS | Squalane | Ethyl oleate | Viscosity (poise) | Availability of formulation |
|---|---------|----------|--------------|-------------------|----------------------------|
| 1 | 670 mg | 8.4 g | 0 | 0.545 | ○ |
| 2 | 670 mg | 6.3 g | 2.1 g | 0.258 | ○ |
| 3 | 670 mg | 4.2 g | 4.2 g | 0.260 | ○ |
| 4 | 670 mg | 2.1 g | 6.3 g | 0.165 | X |
| 5 | 670 mg | 0 | 8.4 g | 0.112 | X |
| 6 | 0 | 8.4 g | 0 | 0.239 | ○ |

In Example 7, the lyophilized formulation of the emulsion was influenced by the viscosity of the paste comprising BCG-CWS used. The paste with viscosity of 0.545 or less provided good lyophilized formulation. Table 8 shows that the paste having too low viscosity fails to provide a good lyophilized formulation. Accordingly, the suitable viscosity of the paste in order to provide a good lyophilized formulation of the emulsion is found to be in the range from0.258 to 0.545.

Example 9

The type of the oil and the availability of the lyophilized formulation

According to a similar procedure to that of Example 7 using oils in Example 2, lyophilized formulation of the emulsion was prepared and evaluated for the availability of the formulation. The results are shown in Table 9.

Table 9

|   | BCG-CWS | Oil (8.4 g) | Viscosity (poise) | Availability of lyophilized formulation |
|---|---------|-------------|-------------------|----------------------------------------|
| 1 | 670 mg | Soybean oil | 6.95 | X |
| 2 | 670 mg | Squalane | 0.55 | ○ |
| 3 | 670 mg | Synthelane 4 | 0.49 | ○ |
| 4 | 670 mg | Drakeol | 0.28 | ○ |
| 5 | 670 mg | Ethyl oleate | 0.11 | X |

As shown in Table 9, it is found that not the type of the oil but the viscosity of the paste comprising BCG-CWS used may have a great influence on the availability of the lyophilized formulation. Referring to Example 8, suitable viscosity of the BCG-CWS paste from which a good lyophilized formulation may be obtained is shown to be 0.28 to 0.55.

Example 10

Preparation Example

BCG-CWS (2640 mg) as cell wall skeleton components was added to a mixture of squalane (35.2 g) and 10 % ethanol/90 % heptane (400 mL), and shaken or treated with ultrasonic at a room temperature to perform dispersion. Then, the dispersion was heated at 60 °C under a flow of nitrogen to evaporate the solvent with stirring. After that, the residue that had been added with 924 g of an aqueous solution of 0.02 %w/w Polysorbate 80 was emulsified with a homogenizer for rough emulsification (7,000 rpm (reversed rotation)/min x 5 minutes), then the resultant was added with 36.6 g of an aqueous solution of 10 %w/w Polysorbate 80 to conduct a complete emulsification (12,000 rpm (normal rotation)/min x 10 minutes). Finally, 1.5 g of a solution of 10 w/w% Polysorbate 80 was added, mixed with stirring (7,000 rpm (reversed rotation) x 1 minute), then the final concentration of Polysorbate was adjusted to 0.4 %w/ w to provide an oil-in-water emulsion. After that, 3,500 g of an aqueous solution of 4 w/w% mannitol was added to provide 4,000 g of the final formulation. (The formulation contains: CWS, 0.6 mg/mL; SQA, 0.8 w/w%; mannitol, 3 w/ w %).

This oil-in-water emulsion was dispensed into 2 mL aliquots in vials, lyophilized to provide lyophilized formulation of the present invention. Lyophilization was carried out using a lyophilizer (GT-6, manufactured by FINTEC, or ULVAC 13A, manufactured by ULVAC).

Example 11

Study on dispersion-aiding solvent:

BCG-CWS (210 mg) was dispersed in various organic solvents (toluene, hexane, heptane, chloroform, dichloroethane, dichloromethane, tetrahydrofuran; 20ml each) by ultrasonication treatment to prepare dispersed drug, then stirred (3,000 rpm x 5 minutes)with a Potter homogenizer (20 ml model), if necessary. The resulting raw material was evaluated for particle size distribution. Moreover, the above raw material solution was mixed with squalane (2.64 g), and the organic solvent was evaporated as described in Example 10 to provide a paste, which was evaluated for particle size distribution.

Particle size distribution of the dispersed raw material was measured using MICRO-TURRAX UPA (manufactured by HANEWEL), and a laser diffraction particle size analyzer (SALD3000, SHIMADZU Corp.) at the concentration of 0.1 mg CWS/mL. The paste was diluted with squalane, and measured using a laser diffraction particle size analyzer (SALD3000, SHIMADZU Corp.) at the concentration of 0.1 - 0.2 mg CWS/mL SQA.

Results

The particle size distribution of the raw material dispersed in toluene was examined, showing two peaks: a sub-micron peak at 400 - 600 nm and a peak of large particle around 10 μm.

The particle size distribution of a mixture obtained by mixing the above drug dispersed in toluene with squalane, and that of a paste prepared by dispersing the above mixture using a Potter type homogenizer were examined. As a result, both of them maintain the particle size distribution similar to those before mixing with squalane.

On the other hand, using hexane, heptane, chloroform, dichloroethane, dichloromethane, tetrahydrofuran and the like as a dispersion-aiding solvent, the particle size distribution around several tens micrometer, as observed in toluene dispersion, was decreased (see Fig. 1). The particle size distribution of BCG-CWS drug in the paste using 90 % heptane/ 10 % ethanol is shown in Fig. 2. As shown in Fig. 2, a homogeneous mixture with a single peak at 0.3 - 2 μm without a sub-peak was obtained.

Table 10 shows the measurements of D 10 %, D90%, and average particle diameter, when the BCG-CWS drugs dispersed in various solvents were measured using a MICRO-TURRAX UPA.

Table 10

| Solvent | D 10 % (μm) | D90% (μm) | Average particle diameter |
|---|---|---|---|
| 10 % ethanol/heptane | 0.23 | 0.60 | 0.41 |
| 5% ethanol/heptane | 0.23 | 0.70 | 0.41 |
| 20 % ethanol/heptane | 0.25 | 0.65 | 0.43 |
| Heptane | 1.19 | 5.07* | 2.90* |

Table 10 (continued)

| Solvent | D 10 % (µm) | D90% (µm) | Average particle diameter |
|---|---|---|---|
| Toluene | 0.33 | 2.08* | 1.03* |

*Since the value of the order of µm has low data precision attributable to the device performance, it is preferred to refer to the value in Table 11.

Table 11 as described below shows the measurements of D 10 %, D90%, and the average particle diameter of BCG-CWS drug dispersed in each solvent as measured by SALD3000.

Table 11

| Solvent | D 10 % (µm) | D90% (µm) | Average particle diameter |
|---|---|---|---|
| 10 % ethanol/heptane | 0.11 | 0.89 | 0.40 |
| Heptane | 0.45 | 1.10 | 0.71 |
| Toluene | 0.39 | 20.86 | 2.65 |
| Toluene | 0.33 | 2.08* | 1.03* |

*Since the value of the order of µm has low data precision attributable to the device performance, it is preferred to refer to the value in Table 11.

Table 12 described below shows the measurements of D 10 %, D90%, and the average particle diameter of BCG-CWS drug dispersed in each solvent as measured by SALD3000.

Table 12

| Solvent | D 10 % (µm) | D90% (µm) | Average particle diameter |
|---|---|---|---|
| 10 % ethanol/heptane | 0.38 | 0.70 | 0.52 |
| Heptane | 0.52 | 5.50 | 1.17 |
| Toluene | 0.39 | 9.91 | 1.05 |

Example 12

Formulation Example

BCG-CWS (1320 mg) as cell wall skeleton components was added to a mixture of squalane (17.6 g) and 10 % ethanol/90 % heptane (200 mL), and shaken or treated with ultrasonic at a room temperature to perform dispersion. Then, the dispersion was heated at 60 - 70 °C under a flow of nitrogen to evaporate the solvent with stirring. After that, the residue that had been added with 462.0 g of an aqueous solution of 0.02 %w/w Polysorbate 80 was emulsified with a homogenizer for rough emulsification (7,000 rpm (reversed rotation)/min x 5 minutes), then the resultant was added with 18.3 g of an aqueous solution of 10 %w/w Polysorbate 80 to conduct a complete emulsification (12,000 rpm (normal rotation)/min x 5 minutes). Finally, 0.76 g of a solution of 10 w/w% Polysorbate 80 was added, mixed and stirred (7,000 rpm (reversed rotation) x 1 minute), then the final concentration of Polysorbate 80 was adjusted to 0.4 %w/w to provide an oil-in-water emulsion. After that, 1,500 g of an aqueous solution of 4 w/w% mannitol was added to provide 2,000 g of the final formulation. (The formulation contains: CWS, 0.6 mg/mL; SQA, 0.8 w/w%; Tween80, 0.1 w/w%; mannitol, 3 w/w %).

This oil-in-water emulsion was dispensed into 2 mL aliquots in vials, lyophilized to provide lyophilized formulation of the present invention. Lyophilization was carried out using a lyophilizer (GT-6, manufactured by FINTEC, or ULVAC 13A, manufactured by ULVAC).

Example 13

Formulation Example

BCG-CWS (2640 mg) as cell wall skeleton components was added to a mixture of squalane (70.4 g) and 10 % ethanol/90 % heptane (400 mL), and shaken or treated with ultrasonic at a room temperature to perform dispersion. Then, the dispersion was heated at 60 - 70 °C under a flow of nitrogen to evaporate the solvent with stirring. After that, the residue that had been added with 888.7 g of an aqueous solution of 0.02 %w/w Polysorbate 80 was emulsified with a homogenizer for rough emulsification (7,000 rpm (reversed rotation)/min x 5 minutes), then the resultant was added with 36.7 g of an aqueous solution of 10 %w/w Polysorbate 80 to conduct a complete emulsification (12,000 rpm (normal rotation)/min x 10 minutes). Finally, 1.5 g of a solution of 10 w/w% Polysorbate 80 was added, mixed with stirring (7,000 rpm (reversed rotation) x 1 minute), then the final concentration of Polysorbate 80 was adjusted to 0.4 %w/w to provide an oil-in-water emulsion. After that, 3,000 g of an aqueous solution of 1.2 w/w% Polysorbate 80/6.7 w/w% mannitol was added to provide 4,000 g of the final formulation. (The formulation contains: CWS, 0.6 mg/mL; SQA, 1.6 w/w%; Tween 80, 1 w/w%; mannitol, 5 w/w %).

This oil-in-water emulsion was dispensed into 2 mL aliquots in vials, lyophilized to provide lyophilized formulation of the present invention. Lyophilization was carried out using a lyophilizer (GT-6, manufactured by FINTEC, or ULVAC 13A, manufactured by ULVAC).

Example 14

Formulation Example

BCG-CWS (1320 mg) as cell wall skeleton components was added to a mixture of squalane (35.2 g) and 10 % ethanol/90 % heptane (200 mL), and shaken or treated with ultrasonic at a room temperature to perform dispersion. Then, the dispersion was heated at 60 - 70 °C under a flow of nitrogen to evaporate the solvent with stirring. After that, the residue that had been added with 444.3 g of an aqueous solution of 0.02 %w/w Polysorbate 80 was emulsified with a homogenizer for rough emulsification (7,000 rpm (reversed rotation)/min x 5 minutes), then the resultant was added with 18.4 g of an aqueous solution of 10 %w/w Polysorbate 80 to conduct a complete emulsification (12,000 rpm (normal rotation)/min x 5 minutes). Finally, 0.76 g of a solution of 10 w/w% Polysorbate 80 was added, mixed with stirring (7,000 rpm (reversed rotation) x 1 minute), then the final concentration of Polysorbate 80 was adjusted to 0.4 %w/w to provide an oil-in-water emulsion. After that, 1,500 g of an aqueous solution of 1.2 w w/w% Tween 80/ 6.7 w/w% mannitol was added to provide 2,000 g of the final formulation. (The formulation contains: CWS, 0.60 mg/mL; SQA, 1.6 w/w%; Tween 80, 1 w/w%; mannitol, 5 w/w %).

This oil-in-water emulsion was dispensed into 2 mL aliquots in vials, lyophilized to provide lyophilized formulation of the present invention. Lyophilization was carried out using a lyophilizer (GT-6, manufactured by FINTEC, or ULVAC 13A, manufactured by ULVAC).

Example 15

The change in particle size distribution after lyophilization

The change in the particle size distribution before and after lyophilization was evaluated for the lyophilized squalane 1.6 % formulation (containing 26.7 g of squalane per gram of BCG-CWS; viscosity, 0.43 poise) prepared in Example 12, according to a similar procedure to that of Example 11.

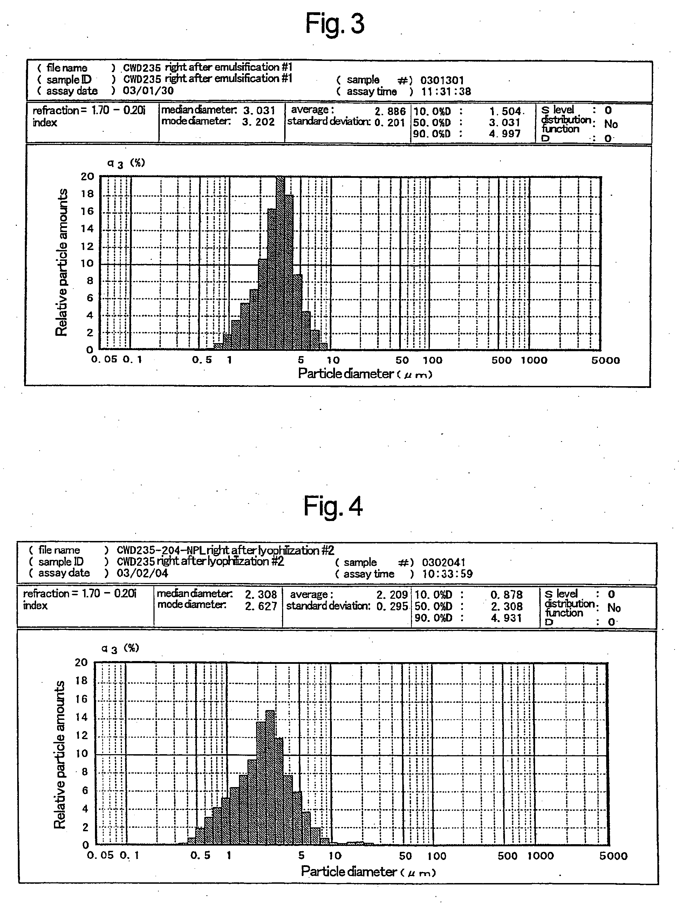

As a result, the lyophilized formulation preserved a single peak particle size distribution with average particle diameter being 2 - 3 μm similar to that of an oil-in-water emulsion before lyophilization (Fig. 3) even after resuspension, wherein 90% D was about 5 μm. On the other hand, the formulation with less squalane content had particles with larger particle diameter (10 μm to 50 μm) mixed therein. In the particle size distribution after lyophilization, the 90 % D value which indicates the existence of larger particle was around 5 μm for 1.6 % formulation.

The above results show that the formulation of Example 12 has highly preserved particle size distribution before and after lyophilization.

Example 16

Change in properties and concentration of drug in suspension after storage of the lyophilized formulation

The squalane 1.6 % formulation (containing 26.7 g of squalane per gram of BCG-CWS; viscosity, 0.43 poise) prepared in Example 12 was evaluated for stability using long-term accelerated storage conditions (5 °C, 25 °C, 40 °C), and severe conditions (60 °C, 80 °C). As the evaluation standard, the concentration of the drug in the suspension described in Example 17 was calculated using a method for determination of mycolic acid, and the state of the suspension was visually evaluated.

As the results, the squalane 1.6 % formulation did not provide generation of adhesion even after storage at 40 °C for 3 months, nor provide decrease in drug concentration in the suspension, as shown in Table 13. Table 14 shows that no generation of adhesion nor decrease of drug concentration in the suspension was observed even after storage under severe conditions (at 60 °C, 80 °C for a month).

The above results indicate that the formulation of Example 12 exhibits excellent storage stability.

Table 13

| Lot | | Initial 5 °C | 25 °C | 40 °C |
|---|---|---|---|---|
| | | x3months | x3months | x3months |
| Squalane 1.6% formulation | 98 (-) | 99 (-) | 100 (-) | 102 (-) |
| *SMP-105 concentration of the suspension is expressed as percentage based on the desired concentration. The amount of adhesion is reported in parentheses. | | | | |

Table 14

| | Lot | Initial | 60 °C x1 month | 80 °C x1 month |
|---|---|---|---|---|
| | Squalane 1.6% formulation | 98 (-) | 102 (-) | 100 (-) |
| *SMP-105 concentration of the suspension is expressed as percentage based on the desired concentration. The amount of adhesion is reported in parentheses. | | | | |

Example 17

Determination of mycolic acid contained in BCG-CWS

(1) Preparation of a solution

1) BCG-CWS was prepared from BCG Tokyo strain (manufactured by Japan BCG) according to the well-known method (J. Nat. Cancer Inst., 52, 95 - 101 (1974)). About 15 g of the test sample of BCG-CWS and a standard BCG-CWS whose biological activity had been validated were weighed precisely, to which was added toluene or a 9:1 mixture of heptane/ethanol (99.5) to bring the volume to exactly 25 mL, then the solvent was evaporated. Similarly, the sample solution (1, 2 and 3 mL) was precisely measured, and the solvent was evaporated.

3) To the test solution and the standard solution was added an internal standard solution (erucic acid (manufactured by Aldrich) in toluene (1g/5000mL) or a tricosenoic acid (manufactured by Tokyo Kasei) in toluene (3g/1000mL)) (precisely 100 μL).

4) 1 mL of a solution containing 0.5 mol/L potassium hydroxide in ethanol (99.5)/toluene/water mixture (10:10:1) was added, then treated with ultrasonic for 1 minute.

5) The resultant was heated in a temperature controlled bath at 65 °C for 3 hours, and after cooling, 2 mol/L hydrochloric acid (precisely 500 μL) was added, and thoroughly mixed by shaking. Further, hexane (2mL) was added, and the resultant was mixed thoroughly by shaking and allowed to stand, then the upper layer (an organic layer) was transferred into a sample bottle. The same procedure was repeated twice using hexane (1mL for each procedure).

6) Water (1 mL) was added to the collected upper layer and after mixing thoroughly and centrifuging, the aqueous layer (lower layer) was removed. The above procedure was repeated once more.

7) The solvent in the upper layer was evaporated using a centrifugal evaporator or nitrogen.

8) The residue was dissolved upon addition of toluene (4 mL), then the resulting solution (200 μL) was precisely weighed into a sample bottle, to which was added toluene (300 μL) and fluorescent labeled test solution (ADAM (9-anthryldiazomethane; manufactured by Funakoshi) in acetone (1g/250 mL)(500 μL), and reacted at 40 °C for 5 hours or more. After completion of the reaction, the resultant was immediately cooled to 5 °C, and analyzed as a test solution and a standard solution, respectively.

(2) Analysis

The test solution and the standard solution (10 µL, each) were analyzed by HPLC using the following conditions. The chromatogram of the test solution exhibiting elution pattern with multiple peaks derived from mycolic acid was compared with that of the standard solution. The peak area ratio of the mycolic acid derivative to that of the internal standard was obtained, and the strength of the sample was obtained using a standard curve plotted from the data of the standard solution.

[Setting of the high performance liquid chromatography (HPLC)]

Column temperature : 50 °C
Column : Mightysil™ RP-18 GP 150-4.6 (5 µm, 4.6 mmΦ x 15 cm) manufactured by Kanto Kagaku, or SUMIPAX ODS A-211 (5 µm, 4.6 mmΦ x 25 cm) manufactured by SUMIKA CHEMICAL ANALYSIS SERVICE, LTD.), or Develosil C30-UG™ (3 µm, 4.6 mmΦ x 15 cm or 5 µm, 4.6 mmΦ x 25 cm) manufactured by Nomura Chemical Co., Ltd.) Solvent: gradient of A: methanol/water mixture (9: 1) and B: 2-propanol, or gradient of A: methanol and B: toluene as shown in Table 15.

Table 15

| Gradient of A: methanol/water mixture (9: 1) and B: 2-propanol | | | | | | |
|---|---|---|---|---|---|---|
| Min | 0 | 25 | 35 | 36 | 39 | |
| A(%) | 90 | 5 | 5 | 90 | 90 | |
| B(%) | 10 | 95 | 95 | 10 | 10 | |
| Gradient of A: methanol and B: toluene | | | | | | |
| Min | 0 | 15 | 30 | 50 | 50.1 | 60 |
| A(%) | 100 | 90 | 50 | 40 | 100 | 100 |
| B(%) | 0 | 10 | 50 | 60 | 0 | 0 |

Flow rate of the mobile phase; 1.0 or 1.5 mL/min
Detection condition of fluorescence
Excitation wavelength: 365 nm
Detection wavelength; 412 nm

(3) Results

Fig. 5 shows a representative chromatogram, Fig. 6 shows a standard curve prepared by plotting the amount of the mycolic acid in the standard measured at several concentrations.
The elution pattern of the mycolic acid in the test sample was compared with that of the standard, and found to exhibit peaks of the same strength at the same position. The strength obtained from the measurements of the test sample using the standard curve in Fig. 6 is shown in Table 16.

Table 16

| Sample (Lot No.) | Titer (%) |
|---|---|
| 0023 | 100 |
| 0104 | 96 |
| 0107a | 98 |
| Titer (%) = (amount of CWS in the sample calculated from the standard curve)/ (amount of sample) x100 | |

As described above, the strength of the BCG-CWS which is an intermediate material for production of the formulation comprising BCG-CWS as an effective ingredient can be assayed.

Example 18

Identification and determination of the bacterial strain

(1) Preparation of the solution

1) About 10 mg (in terms of CWS) of the BCG Tokyo strain (manufactured by Japan BCG), killed by the known method was precisely weighed, to which was added 5 mol/L solution of potassium hydroxide in ethanol (99.5)/water mixture (1:1)(2 mL), and allowed to react at 100 °C for 2 hours. After cooling, the reaction was acidified by addition of 6 mol/L hydrochloric acid (1.5 mL), and mixed by shaking.

2) Separately, about 15 mg of the BCG-CWS standard whose biological activity had been validated was weighed precisely, to which was added toluene or heptane/ethanol (99.5) mixture (9.1) to bring the volume to 25 mL precisely, and treated with ultrasonic for 15 minutes to provide a standard solution. The standard solution (1, 2, and 3 mL) was measured precisely, and the solvent was evaporated. 0.5 mol/L solution of potassium hydroxide in ethanol (99.5)/ toluene/water mixture (10:10:1) (1 mL) was added, and subjected to ultrasonic treatment for 1 minute. The reaction was heated in a temperature controlled bath at 65 °C for 3 hours. After cooling, 2 mol/L hydrochloric acid (500 μL) was added to the reaction and mixed thoroughly by shaking.

3) Hexane (2 mL) was added to the test solution and the standard solution, and mixed thoroughly by shaking and allowed to stand. After that, the upper layer (organic layer) was transferred into a sample bottle. The same procedure was repeated twice using hexane (1 mL for each procedure).

4) Water (1 mL) was added to the collected upper layer, which was mixed thoroughly. After centrifugation, the aqueous layer (lower layer) was removed. The above procedure was repeated one more time.

5) The solvent in the upper layer was evaporated using a centrifugal evaporator or nitrogen.

6) A certain amount of toluene was added to the residue to bring the concentration to about 0.3 mg/mL (in terms of CWS). The solution (200 μL) was measured precisely into a sample bottle, to which were added toluene (300 μL) and fluorescent labeling agent (ADAM (9-Anthryldiazomethane); manufactured by Funakoshi) (500 μL) and allowed to react at 40 °C for 5 hours or longer. After completion of the reaction, the reaction was immediately cooled to 5 °C. Thus obtained products were subjected to analysis as a sample solution and a standard solution, respectively.

(2) Analysis

The sample solution and the standard solution (10 μL, respectively) were analyzed under the same test conditions as those in HPLC of BCG-CWS. The elution pattern exhibiting multiple peaks derived from mycolic acid in the chromatogram of the sample solution was compared with that of the standard solution. Using a peak area of the mycolic acid derivative in the sample solution, and a standard curve prepared from the peak area of the mycolic acid, the content of CWS (%) in the test sample (bacterial strain) was obtained.

(3) Results

The results of the measurement of the test sample are shown in Table 17.

Table 17

| Sample | CWS content (%) |
|---|---|
| Strain 032B | 4.5 |
| Strain 036B | 5.7 |
| Strain 037B | 5.5 |
| Strain 041B | 5.8 |
| CWS content (%) = (the amount of CWS in the sample obtained from the standard curve)/ (the collected amount of the sample) x 100 | |

As mentioned above, an amount of an effective ingredient in each manufacturing lot could be evaluated for BCG Tokyo strain, which is an intermediate material in the production of the pharmaceutical formulations comprising BCG-CWS as an effective ingredient.

Example 19

Analysis of the lyophilized formulation

(1) Preparation of the solution

1) The lyophilized formulation of BCG-CWS was prepared according to the method described in Example 10. About 15 mg of the lyophilized formulation standard of BCG-CWS, whose biological activity had been validated, was precisely weighed, to which was added toluene or heptane/ethanol (99.5) mixture (9: 1) to adjust the volume precisely to 25 mL, which was treated with ultrasonic for 15 minutes to give a standard solution. The standard solution was precisely measured (1, 2 and 3 mL), and the solvent was evaporated. To this standard solution was added an internal standard solution (erucic acid (manufactured by Aldrich) in toluene (1g/5000mL) or a tricosenoic acid (manufactured by Tokyo Kasei) in toluene (3g/1000mL)) (precisely 100 μL), followed by 0.5 mol/L solution of potassium hydroxide in ethanol (99.5)/toluene/water mixture (20:10:1) (1 mL), which was treated with ultrasonic for a minute.
2) The test sample of the lyophilized formulation of BCG-CWS was precisely weighed, to which was added an internal standard solution (100 μL, precisely), followed by 0.5 mol/L solution of potassium hydroxide in ethanol (99.5)/toluene/water mixture (10:10:1) (1 mL), which was treated with ultrasonic for a minute.

After that, hydrolysis was conducted; mycolic acid was extracted with a solvent to perform labeling in the same manner as in Example 1, except that the labeling agent used had concentration 10 times higher than that in Example 1.

(2) Analysis

The sample solution and the standard solution (10 μL, respectively) were analyzed using the following conditions. The elution pattern exhibiting multiple peaks derived from mycolic acid in the chromatogram of the sample solution was compared with that of the standard solution. The peak area ratio of mycolic acid derivative to the internal standard was obtained, and the strength of the test sample was obtained using a standard curve prepared from the sample solution.

(3) Results

The results of the measurement of the test sample are shown in Table 18.

Table 18

| Lot No. | Titer (%) |
|---|---|
| CWD204 | 103 |
| CWD205 | 101 |
| CWD206 | 110 |
| Titer (%) = (The amount of CWS in the formulation obtained from the standard curve)/ (the nominal amount of the drug in the formulation) x 100 | |

As mentioned above, the strength of the pharmaceutical formulation comprising BCG-CWS as an effective ingredient for each lot can be assayed.

Example 20

Identification of the bacterial strain of BCG-CWS

According to the same manner as in Example 1, BCG Tokyo CWS and BCG Pasteur CWS were analyzed for elution pattern of mycolic acid by HPLC. The results are shown in Fig. 7.
As shown in Fig. 7, it is apparent that the different strains can be discriminated from their chromatogram since different types of BCG strains have different type and different ratio of mycolic acid.

Reference Example 1 Biological activity test of BCG-CWS standard

(1) Method

RAW 264.7 cells were seeded in a 96-well plate, and cultivated at 37 °C under conditions (5 % $CO_2$, wet) for 5 hours, and after addition of different concentrations of suspensions of BCG-CWS (standard) in physiological saline, the resultant was further cultured for about 15 hours. The amount of TNF-$\alpha$ in the culture supernatant was subjected to ELISA assay using AN'ALYZA™ Mouse TNF-$\alpha$ Immunoassay kit. According to the accompanied instructions, absorbance at 450 nm was measured. The content of the mycolic acid in the above suspension of BCG-CWS in physiological saline was measured according to the method described in Example 17.

(2) Results

Fig. 8 shows plot of the measured content of mycolic acid in BCG-CWS and the amount of TNF-$\alpha$.

The above results show that, in the BCG-CWS standard, the content of mycolic acid obtained by the present method correlates with the actual biological activity (TNF-$\alpha$ production induction activity). That is, the content of mycolic acid can be used as an index for strength

EFFECTS OF THE INVENTION

The present invention may provide a stable oil-in-water emulsion formulation. The present invention also provides a stable and lyophilizable oil-in-water emulsion formulation and lyophilized formulation thereof comprising solid components in oil. Accordingly, BCG-CWS formulation may be prepared, and immunopotentiating anti-tumor therapy can be more effectively performed using the present method.

According to the present invention, genera, species and strains of bacteria in the bacteria-CWS and the formulation comprising the bacteria-CWS can be identified and determined from the chromatogram pattern of a long-chain fatty acid and a derivative thereof contained in bacteria-CWS of Mycobacterium, Nocardia and the like. The strength of the production intermediate material comprising bacteria-CWS of Mycobacterium, Nocardia and the like can be quantitatively assayed. Thus, the formulation comprising bacteria-CWS of Mycobacterium, Nocardia and the like can be prepared while maintaining the quality of the formulation comprising the bacteria-CWS of Mycobacterium, Nocardia and the like as effective ingredients.

**Claims**

1. A paste comprising bacteria-CWS which consists of a bacteria-CWS and an oil wherein the paste has a viscosity of 0.7 poise or less (25 °C).

2. The paste comprising bacteria-CWS according to claim 1 wherein the paste has a viscosity between 0.2 and 0.7 poise (25 °C).

3. The paste comprising bacteria-CWS according to claim 1 wherein the paste has a viscosity between 0.28 and 0.55 poise (25 °C).

4. The paste comprising bacteria-CWS according to any one of claims 1 to 3 wherein the bacteria-CWS is BCG-CWS.

5. The paste comprising bacteria-CWS according to any one of claims 1 to 4, wherein the oil is squalane.

6. The paste comprising bacteria-CWS, wherein the bacteria-CWS is BCG-CWS and wherein the paste comprises 6.6 g to 35.2 g of squalane per about 0.67 g of BCG-CWS.

7. A process for preparation of a paste comprising bacteria-CWS, which comprises the following steps:

   (1) a step of mixing the bacteria-CWS and oils in an organic solvent used as a dispersion-aiding solvent; and
   (2) a step of removing the organic solvent in (1) by distillation.

8. The process for preparation according to claim 17 wherein the organic solvent comprises a hydrocarbon solvent and a halogenated hydrocarbon solvent.

9. The process for preparation according to claim 8, wherein the organic solvent is a hydrocarbon solvent which comprises 5 to 20 % (v/v) of an alcohol solvent.

10. The process for preparation according to claim 8 or 9, wherein the hydrocarbon solvent is heptane or hexane.

11. A paste comprising bacteria-CWS obtainable by the process for preparation according to any one of claims 7 to 10.

12. The paste according to claim 11. wherein the bacterium is BCG bacteria.

13. The paste according to any one of claims 7 to 12 wherein the oil is squalane.

14. An oil-in-water emulsion which comprises the paste comprising bacteria-CWS according to any one of claims 1 to 6 and 11 to 13, a surfactant, a stabilizer, and water.

15. The oil-in-water emulsion according to claim 14, which comprises 0.66 g to 3.35 g of the bacteria-CWS, and 0.4 wt% to 8 wt% of the oil per 2L of water.

16. The oil-in-water emulsion according to claim 14 or 15, wherein the stabilizer comprises 1 to 10 % mannitol.

17. The oil-in-water emulsion according to any one of claims 14 to 16, wherein the surfactant comprises 0.01 % to 3% polyethyleneoxysorbitan fatty acid ester.

18. The oil-in-water emulsion according to claim 17, wherein the polyethyleneoxysorbitan fatty acid ester is Tween 80.

19. The oil-in-water emulsion according to any one of claim 14 to 18, having the following properties:

    (1) the particle diameter of an oil droplet of the emulsion is 0.2 to 30 $\mu$m;
    (2) the bacteria-CWS is encapsulated in the oil droplet, and is negative for reaction with lectin.

20. A process for preparation of the oil-in-water emulsion according to any one of claims 14 to 19, which comprises the following steps:

    (1) a step of emulsifying a mixture comprising the paste comprising bacteria-CWS according to any one of claims 1 to 10 and 17 to 19, and an aqueous solution containing a surfactant at a temperature higher than the turbidity point; and
    (2) a step of adding an aqueous solution containing a stabilizer for dilution.

21. The process for preparation according to claim 20 wherein the emulsification step in above step (1) comprises the following steps:

    (3) a step of emulsifying a mixture comprising the paste comprising bacteria-CWS according to any one of claims 1 to 6 and 11 to 13, and an aqueous solution containing 0.02 % to 0.8 % of a surfactant (rough emulsification step); and
    (4) a step of adding an aqueous solution containing a surfactant to the mixture of (3) to adjust the concentration of the surfactant, and vigorously stirring the mixture (complete emulsification).

22. A lyophilized formulation obtainable by lyophilizing the emulsion according to any one of claims 14 to 19.

23. The lyophilized formulation according to claim 22, wherein the emulsion is obtainable by the process according to claim 20 to 21.

24. An assembly of bacteria-CWS particles wherein the particle diameter is 0.15 to 6 $\mu$m in the particle size distribution.

25. The assembly of bacteria-CWS particles according to claim 24, wherein the particle size distribution shows a single peak, as well as D 10%: 0.23 $\pm$ 0.05 and D90%: 0.60 $\pm$ 0.05.

26. A process for preparation of the assembly of bacteria-CWS particles according to claim 24 or 25, which comprises dispersing the bacteria-CWS in a solvent containing an aliphatic hydrocarbon solvent.

**27.** The process according to claim 26, wherein the solvent is a mixture of an aliphatic hydrocarbon solvent and an alcohol solvent.

**28.** The process for preparation according to claim 27, wherein the solvent is a heptane containing 5 to 20 % ethanol.

**29.** A process for identification of species and/or strains of a bacterium from which bacteria-CWS is derived, which comprises the following steps:

(1) a step of separating and/or extracting the long-chain fatty acid contained in the bacteria-CWS to prepare a long-chain fatty acid fraction, and if necessary, converting the long-chain fatty acid in the long-chain fatty acid fraction into a derivative thereof;
(2) a step of determining the long-chain fatty acid or a derivative thereof in the long-chain fatty acid fraction of (1) by chromatography; and
(3) a step of identifying species and strains of a bacterium from which the bacteria-CWS is derived based on the results of determination (2).

**30.** The process according to claim 29 wherein step (1) comprises a step of labeling the long-chain fatty acid in the long-chain fatty acid fraction to prepare a labeled long-chain fatty acid derivative;

**31.** A process for assay of the strength of a bacteria-CWS, which comprises the following steps:

(1) a step of separating and/or extracting the long-chain fatty acid contained in the bacteria-CWS to prepare a long-chain fatty acid fraction, and if necessary, converting the long-chain fatty acid in the long-chain fatty acid fraction into a derivative thereof;
(4) a step of determining the content of the long-chain fatty acid or a derivative thereof in the long-chain fatty acid fraction; and
(5) a step of evaluating for an immunopotentiating activity of the bacteria-CWS based on the results of determination (4).

**32.** The process according to claim 31, wherein step (1) determining the content of the long-chain fatty acid or a derivative thereof comprises a step of labeling the long-chain fatty acid in the long-chain fatty acid fraction to prepare a labeled long-chain fatty acid derivative.

**33.** The process according to claim 30 or 32, wherein a derivative of the long-chain fatty acid is a long-chain fatty acid ester.

**34.** The process according to any one of claims 29 to 33, wherein the bacteria are those of Mycobacterium or Nocardia.

**35.** The process according to claim 34, wherein the bacteria of Mycobacterium are those of BCG.

**36.** The process according to any one of claims 29 to 35, wherein the long-chain fatty acid is mycolic acid.

**37.** The paste comprising bacteria-CWS according to claims 1 to 6 and 11 to 13 which comprises an assembly of bacteria-CWS particles, wherein the particle diameter is from 0.1 μm to 20 μm, preferably from 0.15 to 6μm, and more preferably 0.2 μm to 2 μm in the particle size distribution.

**38.** The paste comprising bacteria-CWS according to claim 37, wherein the assembly of bacteria-CWS particles exhibit a particle size distribution showing a single peak as well as D10%: 0.23 ± 0.05 and D90%: 0.60 ± 0.05.

**39.** An oil-in-water emulsion which comprises the paste comprising bacteria-CWS according to claim 37 or 38, a surfactant, a stabilizer, and water.

**40.** A lyophilized formulation obtainable by lyophilizing the emulsion according to claim 39.

**41.** A pharmaceutical composition which consists of the emulsion according to any one of claims 14 to 19 and 39.

## Fig. 1

| S level : 0 distribution : No D shift: 0 function | 90.0%D : 5.498    50.0%D : 0.680    10.0%D : 0.465 |
|---|---|
| | median diameter : 0. 680  mode diameter : 0. 657 |
| q3(%) | refraction index = 1. 70-0. 20i | average : 0. 863  standard deviation : 0. 361 |

## Fig. 2

| S level : 0 distribution : No D shift: 0 function | 10.0%D : 0.379    50.0%D : 0.520    90.0%D : 0.695 |
|---|---|
| | median diameter :0. 520  mode diameter :0. 538 |
| q3(%) | refraction index = 1. 70-0. 20i | average :0. 521  standard deviation :0. 105 |

29

EP 1 547 607 A1

## Fig. 3

| ( file name ) CWD235 right after emulsification #1 | | | | |
| ( sample ID ) CWD235 right after emulsification #1 ( sample #) 0301301 | | | | |
| ( assay date ) 03/01/30 ( assay time ) 11:31:38 | | | | |

| refraction = 1.70 − 0.20i index | median diameter: 3.031 mode diameter: 3.202 | average : 2.886 standard deviation: 0.201 | 10.0%D : 1.504 50.0%D : 3.031 90.0%D : 4.997 | S level : 0 distribution function : No D : 0 |
|---|---|---|---|---|

Particle diameter (μm)

## Fig. 4

| ( file name ) CWD235-204-NPL right after lyophilization #2 | | | | |
| ( sample ID ) CWD235 right after lyophilization #2 ( sample #) 0302041 | | | | |
| ( assay date ) 03/02/04 ( assay time ) 10:33:59 | | | | |

| refraction = 1.70 − 0.20i index | median diameter: 2.308 mode diameter: 2.627 | average : 2.209 standard deviation: 0.295 | 10.0%D : 0.878 50.0%D : 2.308 90.0%D : 4.931 | S level : 0 distribution function : No D : 0 |
|---|---|---|---|---|

Particle diameter (μm)

# Fig. 5

# Fig. 6

Standard curve

Peak area ratio

Amount of CWS (mg)

$y = 106.94x - 0.1703$

$R^2 = 0.9993$

# Fig. 7

# Fig. 8

| **INTERNATIONAL SEARCH REPORT** | International application No. |
|---|---|
| | PCT/JP03/09801 |

**A. CLASSIFICATION OF SUBJECT MATTER**
Int.Cl⁷ A61K35/74, A61K39/39, A61K39/04, A61K39/02, A61K9/107,
A61K9/19, A61K47/06, A61K47/10, A61K47/32, A61K35/00,
C12Q1/04, A61P35/00, A61P37/04
According to International Patent Classification (IPC) or to both national classification and IPC

**B. FIELDS SEARCHED**

Minimum documentation searched (classification system followed by classification symbols)
Int.Cl⁷ A61K35/74, A61K39/39, A61K39/04, A61K39/02, A61K9/107,
A61K9/19, A61K47/06, A61K47/10, A61K47/32, A61K35/00,
C12Q1/04, A61P35/00, A61P37/04

Documentation searched other than minimum documentation to the extent that such documents are included in the fields searched

Electronic data base consulted during the international search (name of data base and, where practicable, search terms used)
CAPlus(STN), BIOSIS(STN), MEDLINE(STN), EMBASE(STN), WPI, JOIS

**C. DOCUMENTS CONSIDERED TO BE RELEVANT**

| Category* | Citation of document, with indication, where appropriate, of the relevant passages | Relevant to claim No. |
|---|---|---|
| X | WO 00/3724 A1 (HAYASHI, A.), 27 January, 2000 (27.01.00), Full text; page 13, 5th line from the bottom to the last line; examples 1.3, 3.3, 3.2; page 23, table 6 & EP 1097715 A1 | 1-28,37-41 |
| X | WO 90/14837 A1 (CHIRON CORP.), 28 November, 1990 (28.11.90), | 1-21,37-39, 41 |
| Y | Full text; page 44, table 8 & EP 399843 A1 & CA 2017507 A1 & JP 5-508385 A & US 6299884 A | 22-28,40 |

☒ Further documents are listed in the continuation of Box C. ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 04 November, 2003 (04.11.03) | 25 November, 2003 (25.11.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

**INTERNATIONAL SEARCH REPORT**

| International application No. |
| --- |
| PCT/JP03/09801 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | WO 99/30737 A1 (CHIRON CORP.),<br>24 June, 1999 (24.06.99),<br>& AU 98/62538 B  & EP 1042001 A1<br>& US 6306405 A  & US 2002/25329 A<br>& JP 2002-508334 A  & US 6458370 A<br>& US 2003/82213 A | 1-28,37-41 |
| Y | WO 00/50006 A1 (CHIRON CORP.),<br>31 August, 2000 (31.08.00),<br>& AU 2000/28757 B  & EP 1156781 A1<br>& JP 2002-537102 A | 1-28,37-41 |
| Y | WO 99/12565 A1 (SMITHKLINE BEECHAM BIOLOGICALS),<br>18 March, 1999 (18.03.99),<br>& AU 9896238 B  & EP 1009430 A1<br>& JP 2001-515870 A  & US 6372227 A<br>& US 2002/58047 A | 1-28,37-41 |
| Y | WO 99/11241 A1 (SMITHKLINE BEECHAM BIOLOGICALS),<br>11 March, 1999 (11.03.99),<br>& AU 9911456 B  & EP 1009382 A1<br>& JP 2001-514208 A  & EP 1279401 A1<br>& US 2003/95974 A | 1-28,37-41 |
| Y | WO 98/56414 A1 (SMITHKLINE BEECHAM BIOLOGICALS),<br>17 December, 1998 (17.12.98),<br>& AU 98/83365 B  & EP 999852 A1<br>& JP 2002-504106 A | 1-28,37-41 |
| Y | WO 95/17210 A1 (SMITHKLINE BEECHAM BIOLOGICALS),<br>29 June, 1995 (29.06.95),<br>& EP 735898 A1  & JP 9-506887 A<br>& AU 687494 B  & US 6146632 A | 1-28,37-41 |
| Y | WO 93/18150 A1 (CHIRON CORP.),<br>16 September, 1993 (16.09.93),<br>& AU 9336300 B  & EP 643770 A1<br>& JP 7-504565 A  & US 5928865 A<br>& EP 967279 A1  & US 6077706 A<br>& US 6090611 A  & US 6130059 A<br>& JP 2000-333686 A  & JP 2000-350591 A<br>& CN 2383007 A1 | 1-28,37-41 |
| Y | WO 00/18431 A1 (CORIXA CORP.),<br>06 April, 2000 (06.04.00),<br>& AU 9962853 B  & US 6171590 A<br>& US 2001/6942 A  & JP 2002-525338 A | 1-28,37-41 |
| Y | WO 96/17863 A1 (IDEC PHARM.CORP.),<br>13 June, 1996 (13.06.96),<br>& AU 9644104 B  & EP 801656 A1<br>& US 5695770 A  & US 5709860 A<br>& JP 10-510264 A | 1-28,37-41 |

Form PCT/ISA/210 (continuation of second sheet) (July 1998)

**INTERNATIONAL SEARCH REPORT**

| International application No. |
| --- |
| PCT/JP03/09801 |

C (Continuation).  DOCUMENTS CONSIDERED TO BE RELEVANT

| Category* | Citation of document, with indication, where appropriate, of the relevant passages | Relevant to claim No. |
| --- | --- | --- |
| X | FLOYD, M.M. et al., 'Separation of Mycobacteriumu bovis BCG from Mycobacterium tuberculosis and Mycobacterium bovis by using high-performance liquid chromatography of mycolic acids.', J.Clin. Microbiol., 1992, Vol.30, No.5, pages 1327 to 1330 | 29,30 |
| X | DUFFEY, P.S. et al., 'Improved rapid identification of mycobacteria by combining solid-phase extraction with high-performance liquid chromatography analysis of BACTEC cultures.', J.Clin.Microbiol., 1996, Vol.34, No.8, pages 1939 to 1943 | 29,30 |
| X | RYLL, R. et al., 'Immunological properties of trehalose dimycolate (cord factor) and other mycolic acid-containing glycolipids a review.', Microbiol.Immunol., 2001, Vol.45, No.12, pages 801 to 811 | 31-36 |
| X | SUEOKA, E. et al., 'Activation of protein kinase C by Mycobacterial cord factor, trehalose 6-monomycolate. resulting in tumor necrosis factor-$\alpha$ release in mouse lung tissues.', Jpn.J.Cancer Res., 1995, Vol.86, No.8, pages 749 to 755 | 31-36 |
| X | JP 1-207236 A  (Sawai Pharmaceutical Co., Ltd.), 21 August, 1989 (21.08.89), Full text (Family: none) | 31-36 |
| X | Chem.Abstr., Vol.119, 1993(Columbus OH, USA), the abstract No.268762, ASANO, M. et al., 'Endogeneous gamma interferon is essential in granuloma formation induced by glycolipid-containing mycolic acid in mice.', Infection and Immunity, 1993, Vol.61, No.7, pages 2872 to 2878 | 31-36 |
| X | GB 1572368 A  (AZUMA I.), 30 July, 1980 (30.07.80), & JP 53-3514 A          & FR 2355506 A1 | 31-36 |
| X | JP 2002-179577 A  (Kabushiki Kaisha Kurabu Cosmetics), 26 June, 2002 (26.06.02), (Family: none) | 31-36 |

Form PCT/ISA/210 (continuation of second sheet) (July 1998)

## INTERNATIONAL SEARCH REPORT

International application No.

PCT/JP03/09801

| Box I | Observations where certain claims were found unsearchable (Continuation of item 2 of first sheet) |

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box II | Observations where unity of invention is lacking (Continuation of item 3 of first sheet) |

This International Searching Authority found multiple inventions in this international application, as follows:
(See extra sheet.)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.

☐ No protest accompanied the payment of additional search fees.

Form PCT/ISA/210 (continuation of first sheet (1)) (July 1998)

36

## INTERNATIONAL SEARCH REPORT

International application No.

PCT/JP03/09801

Continuation of Box No. II of continuation of first sheet(1)

(1) Claims 1 to 5, 6, 7 to 10, 11 to 19, 20, 21, 22, 23 and 37 to 41 relate to any or a paste containing a bacterial CWS and an oil such as squalane, a method of preparing it, an oil-in-water type emulsion obtained by adding a surfactant, a stabilizer and water to the paste, emulsifying and diluting, a freeze-dried product obtained by freeze-drying the emulsion and a medicinal composition comprising the emulsion.

(2) Claims 24 to 25 and 26 to 28 relate to a mass of bacterial CWS particles having a particle size of 0.15 to 6 µm or a method of preparing it.

(3) Claims 29 and 31 relate to a method of identifying the species and/or strain of a bacterial CWS by analyzing a higher fatty acid or its derivative in the bacterial CWS by chromatography.

(4) Claims 31 to 36 relate to a method of calibrating the titer of a bacterial CWS by analyzing the content of a higher fatty acid or its derivative in the bacterial CWS.

Thus, there is no technical feature (other than having a bacterial CWS as a specific matter) common to a combination of two groups among the inventions groups (1) to (4) differing from each other. Further, it cannot be considered that all of the above combinations have a single problem to be solved which had been unsolved before the priority date of the present case.

Concerning the invention groups (1) and (2), furthermore, a paste comprising BCG-CWS, squalane and an organic solvent and inferred as having the viscosity as specified in claim 1 of the present case, an emulsion prepared from this paste and a freeze dried product had been already known as reported by, for example, the following document cited in the international search report:

WO 00/3724 A1 (HAYASHI, A.),
27 January, 2000 (27.01.00),
Full text; page 13, 5th line from the bottom
to the last line; examples 1.3, 3.3, 3.2;
page 23, table 6
& EP 1097715 A1

Concerning the invention groups (3) and (4), it had been already known to identify the species and/or strain of a bacterial CWS or examine the immunopotentiation ability (for example, inducing TNF-α) of the bacterial CWS by analyzing mycolic acid or its derivative in the bacterial CWS as reported by, for example, the following documents:

FLOYD, M.M. et al., 'Separation of Mycobacteriumu bovis BCG from Mycobacterium tuberculosis and Mycobacterium bovis by using high-performance liquid chromatography of mycolic acids.', J.Clin. Microbiol., 1992, Vol.30, No.5, pages 1327 to 1330

RYLL, R. et al., 'Immunological properties of trehalose dimycolate (cord factor) and other mycolic acid-containing glycolipids a review.', Microbiol.Immunol., 2001, Vol.45, No.12, pages 801 to 811

Form PCT/ISA/210 (extra sheet) (July 1998)

**INTERNATIONAL SEARCH REPORT**

International application No.

PCT/JP03/09801

Continuation of Box No. II-1 of continuation of first sheet(1)

Such being the case, the invention groups (1) to (4) are not considered as having any special technical feature in common and, therefore, are not regarded as a group of inventions so linked as to form a single general inventive concept.

Form PCT/ISA/210 (extra sheet) (July 1998)